# EUROPEAN PATENT APPLICATION

(11) **EP 4 011 381 A1**
(43) Date of publication of application: **15.06.2022**
(21) Application number: 21208527.8
(22) Date of filing: 05.06.2017
(51) Int. Cl.: A61K 35/17, A61K 38/17, C12N 5/0783, A61P 35/02, A61K 39/00, C07K 14/725

(54) **ADOPTIVE CELL THERAPIES AS EARLY TREATMENT OPTIONS**

(30) Priority: 03.06.2016 US 201662345682 P
(62) Divisional of application: 17807666.7
(71) Applicant: Memorial Sloan-Kettering Cancer Center, New York, NY 10065 (US)
(72) Inventor: PARK, Jae, H., New York, 10065 (US); RIVIERE, Isabelle, New York, 10024 (US); WANG, Xiuyan, New York, 10065 (US); PURDON, Terence, Jersey City, 07307 (US); SADELAIN, Michel, New York, 10024 (US); BRENTJENS, Renier, J., New York, 10065 (US)
(74) Representative: Barker Brettell LLP

(57) **Abstract**

Provided are methods and compositions for providing early cell therapy options to subjects, offering the potential benefit of avoiding higher risk treatment modalities.

To be accompanied, when published, by Figure 1 of the drawings.

## Description

### CROSS-REFERENCE TO RELATED APPLICATION

This application claims priority to U.S. Provisional Application Serial No. 62/345,682 filed on June 3, 2016, the content of which is incorporated in its entirety, and to which priority is claimed.

### TECHNICAL FIELD

The present invention relates to the use of early adoptive cell therapy in the management of diseases, such as cancer, offering the treated subject the possibility of avoiding higher risk treatment modalities.

### BACKGROUND

Various immunotherapy and/or cell therapy methods are available for treating diseases and conditions. For example, adoptive cell therapies (including those involving the administration of cells expressing chimeric receptors specific for a disease or disorder of interest, such as chimeric antigen receptors (CARs) and/or other recombinant antigen receptors, as well as other adoptive immune cell and adoptive T cell therapies) can be effective in the treatment of cancer and other diseases and disorders. Alternative methods are needed, for example, to provide increased efficacy in certain patient populations and/or for treating certain diseases or conditions. Among the provided embodiments are methods and uses that address such needs.

### SUMMARY OF THE DISCLOSED SUBJECT MATTER

Provided are methods and compositions for treatment of subjects having or suspected of having a disease or condition, such as cancer or other proliferative disorder. In some embodiments, the cancer is a lymphoma or leukemia, such as a B cell proliferative disorder or malignancy such as a CD19+ B cell malignancy. In some embodiments, the methods involve administering to the subject a dose of cells expressing a recombinant antigen receptor (alternatively referred to herein as "RAR cells"). In some embodiments, the cells are T cells expressing a recombinant antigen receptor. In some embodiments, the recombinant antigen receptor may be an immune receptor such as a T cell receptor or chimeric antigen receptor. In some embodiments, the receptor is a chimeric receptor. In some embodiments, the chimeric antigen receptor binds to CD19.

In some embodiments, the administration of the RAR cells is used as a first-line therapy and/or the subject has not received a prior treatment for the disease or condition or has not received such a treatment other than induction chemotherapy and/or consolidating chemotherapy, and/or has not received such therapy since diagnosis of the disease or condition. In some embodiments, the patient is not further administered another therapy, e.g. another cell-mediated therapy such as a hematopoietic stem cell transplant (HSCT), in some cases even if the patient has undergone remission following the administration and/or is otherwise a candidate for HSCT.

In some embodiments, the methods are suitable for treatment of patients without prior, concurrent, simultaneous, or subsequent treatment with one or more other therapies and/or who are not candidates for such one or more other therapies. In some aspects, the subject does not subsequently or simultaneously receive a hematopoietic stem cell transplant (HSCT), or does not go on to do so within a certain period of time, such as 1 month, 2, months, 3 months, 4 months, 5 months, 6 months, 9 months or 1 year subsequent to the administration of the cell dose or the initiation thereof. In some aspects, the subject does not, or is not intended or expected to, receive an HSCT subsequent to the RAR cell dose administration and/or within a certain period of time, such as within 1 month, 2, months, 3 months, 4 months, 5 months, 6 months, 9 months or 1 year, within the administration of the RAR cells or initiation of RAR cell treatment. In some embodiments, the methods do not involve the administration of HSCT and/or ablative irradiation or chemotherapy and/or other therapy aside from doses of the RAR cells and/or optionally aside from induction and/or consolidating chemotherapy.

In some aspects, the subject has not previously been treated with an HSCT and/or has not been treated with an HSCT since diagnosis with the disease or condition and/or within the last year or two years or six months.

In some embodiments, the subject is naive for HSCT (i.e., HSCT-naive) and/or is naive for one or more other treatments for the disease or condition currently subject to treatment, and/or is naive for cancer therapy or chemotherapy or cytoreductive chemotherapy. In some embodiments, an HSCT-naive subject (or subject naive for the other therapy) is one that has not ever received a hematopoietic stem cell transplant (or other therapy) and/or has not received it since diagnosis of or since acquiring the disease or condition currently subject to treatment. In some embodiments, the subject is maintained in such naive status for a certain period of time following the administration of the RAR cell dose or initiation of RAR cell therapy, such as for at least 1, 2, 3, 4, 5, 6, 9, or 12 months following the initiation of the administration of the first dose of RAR cells. In some embodiments, the subject has not been administered a HSCT prior to the initiation of the administering of the antigen receptor-expressing cells. In some aspects, the subject has not been administered a therapy for the disease or condition prior to the administration of the cells and/or since diagnosis of the disease or condition; the subject has not received a therapy for the disease or condition, other than induction chemotherapy or a prior dose of the cells, prior to the administration and/or since diagnosis of the disease or condition; and/or the subject has not received a therapy for the disease or condition, other than induction and/or consolidating chemotherapy, prior to the administration of the RAR cell dose or since diagnosis of the disease or condition.

In some aspects, the administration of HSCT subsequent to treatment, or initiation of treatment, with RAR cell therapy may be contraindicated. In some embodiments, subsequent HSCT may be considered within a specific period of time subsequent to initiation or termination of cell therapy, e.g., within 1, 2, 3, 4, 5, 6, 7, 8, 9, 10, 11, or 12 months after the administration or initiation of such therapy. In specific embodiments, patients having a cancer with one or more specified properties or attributes and that have received the cell dose or doses according to the method should not receive subsequent HSCT, should not receive subsequent HSCT unless other treatment options have been exhausted and/or may exhibit an increased risk of complication-induced death if receiving subsequent HSCT.

In some embodiments, the methods thereby lead to a result indicative of treatment, such as, for example, complete remission, a clinical remission, relapse-free survival, overall survival, MRD-negative response rate, or other favorable outcome. In some embodiments, the result or factor indicative of treatment is enhanced as compared to that observed in subjects receiving treatment by a method that is identical to the method except that the subject receives HSCT subsequently to or within a month, two months, three months or six months of the administering of the antigen receptor-expressing cells.

In some embodiments, the survival of the subject is enhanced, increased or prolonged compared to a subject having received HSCT subsequent to administering the antigen receptor-expressing cells.

Further provided are methods of treatment wherein the method involves administering to a subject having a disease or condition a dose of cells expressing a recombinant antigen receptor, wherein the subject does not concurrently receive and/or has not received a hematopoietic stem cell transplant (HSCT) subsequently to the initiation of the administering of the antigen receptor-expressing cells and/or prior to the initiation of the administration of the antigen-receptor expressing cells, and/or contemporaneously with the initiation of the administration of the antigen receptor-expressing cells. In some embodiments, the subject has not received an HSCT prior to or contemporaneously with administering the antigen receptor-expressing cells and the subject does not receive an HSCT subsequent to administering the antigen receptor-expressing cells.

Further provided are methods of administering to a subject having a disease or condition a dose of cells expressing a recombinant receptor, wherein the subject does not receive a hematopoietic stem cell transplant (HSCT) subsequent to administering the antigen receptor- expressing cells.

Further provided are methods of administering to a subject having a disease or condition a dose of cells expressing a recombinant receptor, wherein the subject has not received a hematopoietic stem cell transplant (HSCT) prior to or contemporaneously with the administration of the cells. In some embodiments, the disease or condition is a tumor or a cancer. In some embodiments, the disease or condition is selected from the group consisting of leukemia, lymphoma, chronic lymphocytic leukemia (CLL), acute-lymphoblastic leukemia (ALL), non-Hodgkin's lymphoma, acute myeloid leukemia, multiple myeloma, refractory follicular lymphoma, mantle cell lymphoma, indolent B cell lymphoma, B cell malignancies, cancers of the colon, lung, liver, breast, prostate, ovarian, skin, melanoma, bone, and brain cancer, ovarian cancer, epithelial cancers, renal cell carcinoma, pancreatic adenocarcinoma, Hodgkin's lymphoma, cervical carcinoma, colorectal cancer, glioblastoma, neuroblastoma, Ewing sarcoma, medulloblastoma, osteosarcoma, synovial sarcoma, mesothelioma, and combinations thereof. In some specific embodiments, the disease or condition is a leukemia or lymphoma, which is optionally a B cell leukemia or lymphoma. In some embodiments, the subject exhibits morphologic disease at the time of initiation of administration of the antigen receptor-expressing cells.

Further provided are methods of treatment, involving administering to a subject having a cancer exhibiting morphologic disease a dose of cells expressing a recombinant antigen receptor, wherein the subject does not receive a hematopoietic stem cell transplant (HSCT) subsequent to the administration of the antigen receptor-expressing cells. In some embodiments, the subject is selected as having a HSCT naive status at the time of initiation of the administration of antigen receptor-expressing cells. In some embodiments, the subject is selected as having a cancer exhibiting morphologic disease at the time of initiation of the administration of the antigen receptor-expressing cells.

Also provided herein is a method of treatment, comprising:
(a) selecting a subject with a disease or condition who (i) has not (A) received a hematopoietic stem cell transplant (HSCT), (B) received an HSCT since diagnosis of the disease or condition, (C) been administered a therapy for said disease or condition, (D) been administered a therapy for said disease or condition other than induction chemotherapy, (E) been administered a therapy of said disease or condition other than consolidating therapy and/or a prior dose of the cells and/or (ii) is an adult; and/or (iii) exhibits morphologic disease or a disease burden above a certain threshold; and/or (iv) is not a candidate for HSCT; and/or (v) is not intended to or set to receive HSCT therapy; and
(b) administering to the subject a dose of cells expressing a recombinant antigen receptor, which is optionally a chimeric antigen receptor.

In some embodiments, the subject thereby exhibits clinical remission, molecular remission, complete remission, or relapse-free survival at least about 1 month, about 2 months, about 3 months, about 4 months, about 5 months, about 6 months, about 12 months, about 24 months, about 36 months, or about 48 months following the administration of the RAR cells; in a non-limiting subset of embodiments said subject has, within said time period, received no HSCT. In some embodiments, the disease or condition is a tumor or a cancer. In some embodiments, the subject exhibits morphologic disease at the time of initiation of administration of the antigen receptor-expressing cells.

In some embodiments, the methods of treatment provided involve
(a) selecting a subject having a cancer exhibiting morphologic disease;
(b) administering to the subject a dose of cells expressing a recombinant antigen receptor; and
(c) not administering a hematopoietic stem cell transplant (HSCT) to the subject subsequently to administering the antigen receptor-expressing cells.

In some embodiments, the methods of treatment provided involve
(a) selecting a subject having a cancer exhibiting minimal or molecular disease;
(b) administering to the subject a dose of cells expressing a recombinant antigen receptor; and
(c) not administering a hematopoietic stem cell transplant (HSCT) to the subject subsequently to administering the antigen receptor-expressing cells.

In some embodiments, the disease or condition is a leukemia or lymphoma. In some specific embodiments, the disease or condition is selected from chronic lymphocytic leukemia (CLL), acute-lymphoblastic leukemia (ALL), non-Hodgkin's lymphoma and acute myeloid leukemia, and optionally is B cell-derived. In some further embodiments, the disease or condition is a non-Hodgkin's lymphoma (NHL), which optionally is B cell-derived. In other further embodiments, the disease or condition is acute-lymphoblastic leukemia (ALL). In other further embodiments, the disease is B-cell derived acute-lymphoblastic leukemia (ALL).

In some embodiments that may be combined with other embodiments and/or methods disclosed herein, the subject is administered HSCT prior to administering the antigen receptor-expressing cells. In other embodiments, the subject is not administered HSCT prior to administering the antigen receptor-expressing cells and/or has not been administered such a therapy within a certain time period prior to the administration of the cell dose, such as not since diagnosis with the disease or condition and/or not within the past about 1 month, about 2 months, about 3 months, about 4 months, about 5 months, about 6 months, about 7 months, about 8 months, about 9 months, about 10 months, about 11 months, about 12 months or past about 1 year, about 2 years, about 3 years, or about 4 years or more.

In some embodiments, that may be combined with other embodiments and/or methods disclosed herein, morphologic disease comprises greater than 5%, or greater than about 5 %, blast cells in the bone marrow. In certain embodiments, morphologic disease comprises greater than 20%, or greater than about 20%, blast cells in the bone marrow. In some embodiments, the morphologic disease comprises greater than 50%, or greater than about 50%, blast cells in the bone marrow. In some embodiments, minimal or molecular disease involves fewer (less) than about 5% or about 5% blasts in the bone marrow, and/or wherein blasts are essentially undetected in the blood or bone marrow by a certain assay such as flow cytometry assay, but are detectable by molecular methods such as PCR.

In certain embodiments, the subject exhibits the minimal disease and/or is selected as having minimal disease. In some embodiments, at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission.

In some embodiments, at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.

In some embodiments, that may be combined with other embodiments and/or methods disclosed herein, the subject exhibits a cancer comprising molecularly detectable disease at the time of initiation of the administration of the antigen receptor-expressing cells.

In some embodiments, the methods disclosed herein result in enhanced overall survival of the subject compared to a subject having receiving HSCT prior to, contemporaneously with and/or subsequent to the administration of the antigen receptor-expressing cells. In some embodiments, the method results in enhanced overall survival of the subject compared to a subject having received HSCT subsequent to the administration of the antigen receptor-expressing cells.

In some embodiments and/or methods disclosed herein, the average median overall survival among subjects following treatment using the method is enhanced by greater than or greater than about 2 months, greater than or greater than about 6 months, greater than or greater than about 1 year or greater than or greater than about 2 years subsequent to administration of the antigen receptor-expressing cells compared to that among subjects treated with a method that is identical to the method except that it further comprises administering HSCT subsequently and/or prior to the administration of the antigen receptor-expressing cells.

In some embodiments, and/or methods disclosed herein, at least about 50%, at least about 60 %, at least about 70%, at least about 80%, at least about 90%, or at least about 99% of subjects treated with the method exhibit a period of survival, a period of event-free survival, and/or a period of relapse-free survival that is greater than or greater than about 6 months, 1 year, 2 years, greater than or greater than about 3 years, greater than or greater than about 4 years or more following the initiation of the administration of the antigen receptor-expressing cells. In some embodiments, the HSCT is allogeneic to the subject. In some embodiments, at the time of initiation of the antigen receptor-expressing cells the subject has not been previously administered a therapy or therapeutic agent for treating the disease or condition, and/or has not received a therapeutic agent targeting the tumor or cancer and/or has not received any such agent other than another dose of the cells or another dose of cells expressing a chimeric receptor.

In some embodiments, at the time of initiation of administering the antigen receptor-expressing cells the subject has relapsed following administration of a previous therapy or therapeutic agent for treating the disease or condition; or the subject has been treated with therapy or therapeutic agent for treating the disease or condition, optionally a therapeutic agent targeting the tumor or cancer, prior to administering the antigen receptor-expressing cells and is refractory or non-responsive to said therapy or therapeutic agent at the time of initiation of administration of the antigen receptor-expressing cells. In some embodiments, the method results in a reduction in burden of the disease or condition in the subject, as indicated by a reduction in one or more factors indicative of disease burden. In some embodiments, the antigen receptor expressed by the cells specifically binds to an antigen expressed by a cell or tissue of the disease or condition or associated with the disease or condition.

In some embodiments and/or methods disclosed herein, the recombinant antigen receptor is a T cell receptor or a functional non-T cell receptor; and/or the recombinant antigen receptor is a chimeric antigen receptor (CAR). In some embodiments, the recombinant antigen receptor is a CAR. In some embodiments, the antigen receptor comprises an extracellular antigen-recognition domain that specifically binds to the antigen and an intracellular signaling domain comprising an ITAM. In some embodiments the antigen comprises CD19. In some embodiments, the intracellular signaling domain comprises an intracellular domain of a CD3-zeta (CD3ζ) chain. In some embodiments, the CAR further comprises a costimulatory signaling region. In some embodiments, the costimulatory signaling region comprises a signaling domain of a CD28 and optionally a transmembrane and/or extracellular portion of a CD28. In other embodiments, the costimulatory signaling domain comprises a signaling domain of a 4-1BB.

In some embodiments, the chimeric antigen receptor (CAR) is a CD19 directed CAR comprising a signaling domain of CD28 as set forth in international patent application publication number WO 2014134165, WO 2008121420, and U.S. Patent Number 7,446,190.

In some embodiments, the cells into which recombinant antigen receptor is introduced comprise or are T cells. In some embodiments, the T cells are CD4⁺ and/or CD8⁺. In some embodiments, the T cells are autologous to the subject. In some embodiments, the dose of cells comprises cells in an amount sufficient for reduction in burden of a disease or condition in the subject. In some embodiments, the ratio of CD4⁺ to CD8⁺ cells is between about 1:5 and about 5:1. In some embodiments, the ratio of CD4⁺ to CD8⁺ cells is between about 1:2 and about 2:1.

In some embodiments that may be combined with any other embodiment and/or method disclosed herein, the dose of cells comprises between about 0.2 × 10⁶ cells/kg body weight of the subject and about 6 × 10⁶ cells/kg, about 0.5 × 10⁶ cells/kg body weight of the subject and about 3 × 10⁶ cells/kg, between about 0.75 × 10⁶ cells/kg and about 2.5 × 10⁶ cells/kg or between about 1 × 10⁶ cells/kg and about 2 × 10⁶ cells/kg, each inclusive. In some embodiments, the cells of the dose are administered in a single pharmaceutical composition comprising the cells. In some embodiments, the cells of the dose are administered in a plurality of compositions, collectively comprising the cells of the dose, over a period of no more than three days.

In some embodiments, the methods disclosed herein further comprise administering to the subject a consecutive dose of cells expressing a recombinant antigen receptor after administering the first dose of antigen receptor-expressing cells.

In some embodiments, the first and consecutive doses are administered in a manner as described in PCT application No. WO2016/064929. In some embodiments, the consecutive dose of antigen receptor-expressing cells is administered at a time point that is between about 9 and about 35 days, between about 14 and about 28 days, between about 15 and about 27 days or is between about 17 days and about 21 days, each inclusive, after initiation of administration of the first dose of cells.

In some embodiments, the antigen receptor expressed by the cells in the consecutive dose is identical to the antigen receptor expressed by cells in the first dose or is substantially identical to the antigen receptor expressed by cells in the first dose. In some embodiments, the antigen receptor expressed by the cells in the consecutive dose specifically binds to the same antigen as the antigen specifically bound by the antigen receptor expressed by cells of the first dose.

In some embodiments, the consecutive dose of cells comprises cells in an amount sufficient for reduction in burden of a disease or condition in the subject. In some embodiments, the consecutive dose of cells comprises less than or about the same number of antigen receptor-expressing cells as the number of antigen receptor-expressing cells in the first dose. In some embodiments, the subject exhibits morphologic disease after initiation of the administration of the first dose of cells and before initiation of administration of the consecutive dose of cells.

In some embodiments, the consecutive dose comprises an increased number of antigen receptor-expressing cells as compared to the first dose. In some embodiments, the increased number is at least 2-fold, 5-fold, or 10-fold greater than the number in the first dose. In some embodiments, the subject exhibits minimal disease after initiation of the administration of the first dose of cells and before initiation of administration of the consecutive dose of cells.

Aspects or embodiments of the invention may also be provided according to the following paragraphs:
1. A method of treatment comprising:
   (a) administering to a subject having a disease or condition a dose of cells expressing a recombinant antigen receptor, which is optionally a chimeric antigen receptor; and
   (b) not administering a hematopoietic stem cell transplant (HSCT) to the subject subsequently to administering the antigen receptor-expressing cells and/or not administering an HSCT to the subject within about 1 month, about 2 months, about 3 months, about 4 months, about 5 months, about 6 months, or about 1 year subsequently to the initiation of the administration in (a), and/or wherein the subject is not administered an HSCT subsequently to the administration of the cells or within about 1 month, about 2 months, about 3 months, about 4 months, about 5 months, about 6 months, or about 1 year subsequently to the initiation of the administration in (a) and/or, not so-administering HSCT in the absence of relapse of the subject, optionally following a clinical remission.
2. A method of treatment comprising administering to a subject that has a disease or condition and is HSCT-naive a dose of cells expressing a recombinant antigen receptor, which is optionally a chimeric antigen receptor, wherein the subject is thereafter maintained as HSCT-naive for at least about 1, 2, 3, 4, 5, 6, 9, or 12 months following the initiation of the administration of the dose of cells.
3. The method of paragraph 1, wherein the subject has not been administered a HSCT prior to the initiation of the administering of the antigen receptor-expressing cells.
4. The method of any one of paragraphs 1-3, wherein the subject has not been administered a therapy for the disease or condition prior to the administration in (a) and/or since diagnosis of the disease or condition.
5. The method of any one of paragraphs 1-4, wherein the subject has not received a therapy for the disease or condition, other than induction chemotherapy or a prior dose of the cells, prior to the administration in (a) and/or since diagnosis of the disease or condition.
6. The method of any one of paragraphs 1-5, wherein the subject has not received a therapy for the disease or condition, other than induction and/or consolidating chemotherapy, prior to the administration in (a), and/or has since diagnosis of the disease or condition.
7. The method of any one of paragraphs 1-4, wherein the subject has not received a therapy for the disease or condition, other than induction chemotherapy or a prior dose of the cells, prior to the administration in (a) and/or since diagnosis of the disease or condition. mean overall survival of subjects is enhanced compared to that observed in subjects receiving treatment by a method that is identical to the method except that the subject receives HSCT subsequently to or within a month, two months, three months or six months of the administering of the antigen receptor-expressing cells.
8. A method of treatment, comprising administering to a subject having a disease or condition a dose of cells expressing a recombinant antigen receptor, wherein the subject does not receive and/or has not received a hematopoietic stem cell transplant (HSCT) subsequently to the initiation of the administering of the antigen receptor-expressing cells and/or prior to the initiation of the administration of the antigen-receptor cells, and/or contemporaneously with the initiation of the administration of the antigen receptor-expressing cells.
9. The method of paragraph 8, wherein the subject has not received an HSCT prior to or contemporaneously with administering the antigen receptor-expressing cells and the subject does not receive an HSCT subsequent to administering the antigen receptor-expressing cells or within a period of time from initiation of said administering, which period of time is optionally within or within about 1, 2, 3, 4, 5, 6, 7, 8, 9, 10, 11, or 12 months..
10. The method of treatment of any one of paragraphs 1-9, wherein the disease or condition is a tumor or a cancer.
11. The method of treatment any one of paragraphs 1-10, wherein the disease or condition is selected from the group consisting of leukemia, lymphoma, chronic lymphocytic leukemia (CLL), acute-lymphoblastic leukemia (ALL), non-Hodgkin's lymphoma, acute myeloid leukemia, multiple myeloma, refractory follicular lymphoma, mantle cell lymphoma, indolent B cell lymphoma, B cell malignancies, cancers of the colon, lung, liver, breast, prostate, ovarian, skin, melanoma, bone, and brain cancer, ovarian cancer, epithelial cancers, renal cell carcinoma, pancreatic adenocarcinoma, Hodgkin's lymphoma, cervical carcinoma, colorectal cancer, glioblastoma, neuroblastoma, Ewing sarcoma, medulloblastoma, osteosarcoma, synovial sarcoma, mesothelioma, and combinations thereof.
12. The method of any one of paragraphs 1-11, wherein the disease or condition is a leukemia or lymphoma and/or blood cancer, and/or is a B cell leukemia or lymphoma, and/or which optionally as an acute leukemia, a chronic leukemia and/or a non-Hodgkin lymphoma.
13. The method of any one of paragraphs 10-12, wherein the subject exhibits morphologic disease at the time of or immediately prior to initiation of administration of the antigen receptor-expressing cells or wherein the subject exhibits minimal disease or has relapsed at the time of or immediately prior to initiation of administration of the antigen-receptor expressing cells.
14. A method of treatment, comprising administering to a subject having a cancer exhibiting morphologic disease a dose of cells expressing a recombinant antigen receptor, wherein the subject does not receive a hematopoietic stem cell transplant (HSCT) subsequent to the administration of the antigen receptor-expressing cells.
15. The method of any one of paragraphs 1-14, wherein the subject is selected as (a) having a HSCT naive status, being naive status for another treatment or therapy for the disease or condition, and/or as having received an induction phase of chemotherapy and optionally not having received consolidating chemotherapy, at the time of or prior to the initiation of the administration of antigen receptor-expressing cells; or (b) being or being at least about 18, 20, 30, 40, 50, 55, 60, 65, 70, 75, 80, or 85 years of age.
16. The method of any one of paragraphs 1-15, wherein the subject is selected as having a morphologic disease or having minimal disease at the time of initiation of the administration of the antigen receptor-expressing cells.
17. The method of paragraph 16, wherein the subject exhibits the minimal disease and/or is selected as having minimal disease.
18. The method of any one of paragraphs 1-16, wherein at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission.
19. The method of any one of paragraphs 1-16, wherein at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.
20. A method of treatment, comprising:
   (a) selecting a subject with a disease or condition who (i) has not (A) received a hematopoietic stem cell transplant (HSCT), (B) received an HSCT since diagnosis of the disease or condition, (C) been administered a therapy for said disease or condition, (D) been administered a therapy for said disease or condition other than induction chemotherapy, (E) been administered a therapy of said disease or condition other than consolidating therapy and/or a prior dose of the cells and/or (ii) is an adult; and/or (iii) exhibits morphologic disease or a disease burden above a certain threshold; and/or (iv) is not a candidate for HSCT; and/or (v) is not intended to or set to receive HSCT therapy; and
   (b) administering to the subject a dose of cells expressing a recombinant antigen receptor, which is optionally a chimeric antigen receptor.
21. The method of any one of paragraphs 1-20, wherein the subject thereby exhibits clinical remission, molecular remission, complete remission, or relapse-free survival, optionally without an HSCT, or at least about 1, about 2, about 3, about 4, about 5, about 6, about 12, about 24, about 36, or about 48 months following the administration of the cells.
22. The method of any one of paragraphs 1-21, wherein the disease or condition is a tumor or a cancer.
23. The method of paragraph 21 or 22, wherein the subject exhibits morphologic disease at the time of initiation of administration of the antigen receptor-expressing cells.
24. A method of treatment, comprising:
   (a) selecting a subject having a cancer exhibiting morphologic disease or selecting a subject having minimal disease;
   (b) administering to the subject a dose of cells expressing a recombinant antigen receptor; and
   (c) not administering a hematopoietic stem cell transplant (HSCT) to the subject subsequently to administering the antigen receptor-expressing cells.
25. The method of any one of paragraphs 17-24, wherein the disease or condition is a leukemia or lymphoma.
26. The method of any one of paragraphs 1-25, wherein the disease or condition is selected from chronic lymphocytic leukemia (CLL), acute-lymphoblastic leukemia (ALL), non-Hodgkin's lymphoma and acute myeloid leukemia, and/or optionally is B cell-derived.
27. The method of any one of paragraphs 1-26, wherein the disease or condition is a non-Hodgkin lymphoma (NHL), which optionally is B cell-derived.
28. The method of any one of paragraphs 1-26, wherein the disease or condition is acute-lymphoblastic leukemia (ALL), which optionally is B cell-derived.
29. The method of any one of paragraphs 1-26, wherein the disease or condition is chronic lymphocytic leukemia (CLL), which optionally is B cell-derived.
30. The method of any one of paragraphs 24-29, wherein the subject is administered HSCT prior to administering the antigen receptor-expressing cells or has been so administered.
31. The method of any one of paragraphs 24-30, wherein the subject is not administered HSCT prior to administering the antigen receptor-expressing cells.
32. The method of any one of paragraphs 18-31, wherein morphologic disease comprises greater than or greater than about 5% blast cells in the bone marrow and/or the minimal disease comprises less than about 5% blasts in the bone marrow.
33. The method of paragraph 32, wherein the subject exhibits the minimal disease and/or is selected as having minimal disease.
34. The method of any one of paragraphs 1-32, wherein at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission.
35. The method of any one of paragraphs 1-32, wherein at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.
36. The method of any one of paragraphs 20-32, wherein morphologic disease comprises greater than or greater than about 20% blast cells in the bone marrow.
37. The method of any one of paragraphs 20-36, wherein the morphologic disease comprises greater than or greater than about 50% blast cells in the bone marrow.
38. The method of any one of paragraphs 1-15, 22 and 23, wherein the subject exhibits a cancer comprising molecularly detectable disease at the time of initiation of the administration of the antigen receptor-expressing cells
39. The method of any one of paragraphs 6-38, wherein the method results in enhanced overall survival of the subject compared to a subject having received HSCT prior to, contemporaneously with and/or subsequent to the administration of the antigen receptor-expressing cells.
40. The method of any one of paragraphs 6-39, wherein the method results in enhanced overall survival of the subject compared to a subject having received HSCT subsequent to the administration of the antigen receptor-expressing cells.
41. The method of any one of paragraphs 1-40, wherein the average median overall survival among subjects following treatment using the method is enhanced by greater than or greater than about 2 months, greater than or greater than about 6 months, greater than or greater than about 1 year or greater than or greater than about 2 years subsequent to administration of the antigen receptor-expressing cells compared to that among subjects treated with a method that is identical to the method except that it further comprises administering HSCT subsequently and/or prior to the administration of the antigen receptor-expressing cells.
42. The method of any one of paragraphs 1-41, wherein at least about 50%, at least about 60%, at least about 70%, at least about 80%, or at least about 90% of subjects treated with the method exhibit a period of survival, a period of event-free survival, or a period of relapse-free survival that is greater than or greater than about 6 months, about 1 year, about 2 years, greater than or greater than about 3 years, greater than or greater than about 4 years or more following the initiation of the administration of the antigen receptor-expressing cells.
43. The method of any one of paragraphs 1-42, wherein the HSCT is allogeneic to the subject.
44. The method of any one of paragraphs 1-43, wherein at the time of initiation of the antigen receptor-expressing cells the subject has not been previously administered a therapy or therapeutic agent for treating the disease or condition, and/or has not received a therapeutic agent targeting the tumor or cancer and/or has not received any such agent other than another dose of the cells or another dose of cells expressing a chimeric receptor.
45. The method of any one of paragraphs 1-43 wherein:
   at the time of initiation of administering the antigen receptor-expressing cells the subject has relapsed following administration of a previous therapy or therapeutic agent for treating the disease or condition; or
   the subject has been treated with therapy or therapeutic agent for treating the disease or condition, optionally a therapeutic agent targeting the tumor or cancer, prior to administering the antigen receptor-expressing cells and is refractory or non-responsive to said therapy or therapeutic agent at the time of initiation of administration of the antigen receptor-expressing cells.
46. The method of any one of paragraphs 1-45, wherein the method results in a reduction in burden of the disease or condition in the subject, as indicated by a reduction in one or more factors indicative of disease burden.
47. The method of any one of paragraphs 1-46, wherein the antigen receptor expressed by the cells specifically binds to an antigen expressed by a cell or tissue of the disease or condition or associated with the disease or condition.
48. The method of any one of paragraphs 1-47, wherein:
   the recombinant antigen receptor is a T cell receptor or a functional non-T cell receptor; and/or
   the recombinant antigen receptor is a chimeric antigen receptor (CAR).
49. The method of paragraph 48, wherein the recombinant antigen receptor is a CAR
50. The method of paragraph 48 or 49, wherein the antigen receptor comprises an extracellular antigen-recognition domain that specifically binds to the antigen and an intracellular signaling domain comprising an ITAM.
51. The method of any one of paragraphs 47-50, wherein the antigen comprises CD19.
52. The method of paragraph 50 or 51, wherein the intracellular signaling domain comprises an intracellular domain of a CD3-zeta (CD3ζ) chain.
53. The method of any one of paragraphs 49-52, wherein the CAR further comprises a costimulatory signaling region.
54. The method of paragraph 53, wherein the costimulatory signaling domain comprises a signaling domain of a CD28 and optionally a transmembrane and/or extracellular portion of a CD28.
55. The method of paragraph 53, wherein the costimulatory signaling domain comprises a signaling domain of a 4-1BB.
56. The method of any one of paragraphs 1-54, wherein the cells comprise or are T cells, which may be CD4⁺ and/or CD8⁺.
57. The method of paragraph 56, wherein the T cells are autologous to the subj ect.
58. The method of paragraph 56, wherein the ratio of CD4⁺ to CD8⁺ cells is between about 1:5 and about 5:1.
59. The method of paragraph 58, wherein the ratio of CD4⁺ to CD8⁺ cells is between about 1:2 and about 2:1.
60. The method of any one of paragraphs 1-59, wherein the dose of cells comprises cells in an amount sufficient for reduction in burden of a disease or condition in the subject.
61. The method of any one of paragraphs 1-60, wherein the dose of cells comprises between about 0.2 × 10⁶ cells/kg body weight of the subject and about 6 × 10⁶ cells/kg, about 0.5 × 10⁶ cells/kg body weight of the subject and about 3 × 10⁶ cells/kg, between about 0.75 × 10⁶ cells/kg and about 2.5 × 10⁶ cells/kg or between about 1 × 10⁶ cells/kg and about 2 × 10⁶ cells/kg, each inclusive.
62. The method of any one of paragraphs 1-61, wherein the cells of the dose are administered in a single pharmaceutical composition comprising the cells.
63. The method of any one of paragraphs 1-61, wherein the cells of the dose are administered in a plurality of compositions, collectively comprising the cells of the dose, over a period of no more than three days.
64. The method of any one of paragraphs 1-63, comprising administering to the subject a consecutive dose of cells expressing a recombinant antigen receptor after administering the first dose of antigen receptor-expressing cells.
65. The method of paragraph 64, wherein the consecutive dose of antigen receptor-expressing cells is administered at a time point that is between about 9 and about 35 days, between about 14 and about 28 days, between about 15 and about 27 days or is between about 17 days and about 21 days, each inclusive, after initiation of administration of the first dose of cells.
66. The method of paragraph 64 or 65, wherein the antigen receptor expressed by the cells in the consecutive dose is identical to the antigen receptor expressed by cells in the first dose or is substantially identical to the antigen receptor expressed by cells in the first dose.
67. The method of paragraph 64 or 65, wherein the antigen receptor expressed by the cells in the consecutive dose specifically binds to the same antigen as the antigen specifically bound by the antigen receptor expressed by cells of the first dose.
68. The method of any one of paragraphs 64-67, wherein the consecutive dose of cells comprises cells in an amount sufficient for reduction in burden of a disease or condition in the subject.
69. The method of any one of paragraphs 64-68, wherein the consecutive dose of cells comprises less than or about the same number of antigen receptor-expressing cells as the number of antigen receptor-expressing cells in the first dose.
70. The method of paragraph 69, wherein the subject exhibits morphologic disease after initiation of the administration of the first dose of cells and before initiation of administration of the consecutive dose of cells.
71. The method of any one of paragraphs 64-68, wherein the consecutive dose comprises an increased number of antigen receptor-expressing cells as compared to the first dose.
72. The method of paragraph 71, wherein the increased number is at least about 2-fold, about 5-fold, or about 10-fold greater than the number in the first dose.
73. The method of paragraph 71 or 72, wherein the subject exhibits minimal disease after initiation of the administration of the first dose of cells and before initiation of administration of the consecutive dose of cells.
74. The method of paragraph 73 wherein the subject exhibits the minimal disease and/or is selected as having minimal disease.
75. The method of any one of paragraphs 1-74, wherein at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission.
76. The method of any one of paragraphs 1-75, wherein at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.

### BRIEF DESCRIPTION OF THE DRAWINGS

Figure 1. Complete Remission (CR) Rates by Subgroups. Ph+: Philadelphia chromosome positive; CI: confidence interval.
Figure 2. Overall Survival of All Patients by Baseline Disease Burden.
Figure 3. Overall Survival of MRD-CR Patients by Baseline Disease Burden.
Figure 4. Overall Survival of MRD-CR Patients by Baseline Disease Burden and Post CAR-T HSCT.
Table 1. Baseline Patient Characteristics.
Table 2. Complete Remission (CR) Rates.
Table 3. Cytokine Release Syndrome (CRS) Grading System.
Table 3A. Exemplary Grading Criteria for CRS.
Table 4. CRS & Neurological Toxicities By Baseline Disease Burden.

### DETAILED DESCRIPTION

Provided herein are methods of treatment involving the administration of engineered cells to subjects having a disease or condition. The cells generally are engineered to express one or more recombinant antigen receptor, for example a chimeric antigen receptor (CAR).

### Methods and Uses of Cell Therapy with Genetically Engineered Cells

Provided are methods and compositions for use in cell therapy, for the treatment of diseases or conditions, including various cancers and tumors. The methods involve administering engineered cells expressing recombinant receptors (alternatively referred to herein as "RAR cells") designed to recognize and/or specifically bind to molecules associated with the disease or condition to be treated and result in a response, such as an immune response against such molecules upon binding to such molecules. The receptors may include chimeric receptors, e.g., chimeric receptors, such as chimeric antigen receptors (CARs), and other transgenic antigen receptors including transgenic T cell receptors (TCRs).

In some embodiments, the methods are advantageous in their ability to treat subjects having certain diseases or conditions such as cancers (e.g., a B cell leukemia or lymphoma) earlier after diagnosis of the disease or condition and/or before the subject has received one or more therapy or treatment for the disease or condition. For example, in some embodiments, the methods involve administering one or more doses of the RAR cells to a subject within about 1, about 2, about 3, about 4, about 5, about 6, about 7, about 8, about 9, about 10, about 11, about 12 or more months of diagnosis and/or at a time at which the subject has not received a therapy for the disease or condition and/or has not received a particular type of treatment or therapy, such as a hematopoietic stem cell transplant (HSCT), high-dose chemotherapy or radiation, and/or a therapy other than another dose of the cells (and/or other engineered cells) and/or induction chemotherapy or consolidating chemotherapy. In some embodiments, the methods and uses provide first-line therapy for certain diseases and conditions. In some embodiments, the methods avoid the need to administer additional therapies other than the RAR cells to treat the disease or condition (e.g., cancer), such as other therapies that may carry a risk of side effects (in some cases, particularly in certain subject populations), such as HSCT and/or other therapies. In some embodiments, the methods involve selecting a subject for treatment based on his or her not having been previously treated with one or more given therapies and/or having one or more properties, such as those which may increase the risk of the subsequent administration of such one or more other therapies. In some embodiments, the subject is selected based on age (for example, selection of an adult subject or a subject that is at least 18, 20, 30, 40, 50, 55, 60, 65 70, 75, or 80 years of age), disease burden (for example, selection of a subject having high morphologic disease or minimal disease or molecular disease; a subject is selected to exhibit minimal disease if the subject exhibits less than about 5% blasts in the bone marrow, and in certain embodiments, the subject exhibits molecularly detectable cancer). In some embodiments, the subject exhibits the minimal disease and/or is selected as having minimal disease. In some embodiments, at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission. In some embodiments, at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.

In some embodiments, the subject is selected based on disease or condition, such as leukemia or lymphoma, such as a B cell leukemia or lymphoma and/or acute lymphoblastic leukemia (ALL). In some embodiments, the disease or condition is non-Hodgkin's lymphoma (NHL) and/or chronic lymphocytic leukemia, such as of a B cell origin.

In some embodiments, the methods involve treating a subject with a dose of the RAR cells in which such cells express a recombinant antigen receptor that recognizes or binds to an antigen or molecule associated with the disease or condition and not further administering to the subject a hematopoietic stem cell transplantation (HSCT), e.g., allogenic HSCT, and/or where the subject does not receive such further administration or does not receive it within a certain period of time after the administration of the cell dose or initiation thereof, e.g., within about 1, about 2, about 3, about 4, about 5, about 6, about 7, about 8, about 9, about 10, about 11, or about 12 months after the administration or initiation of such cell dose, which may be a first or subsequent (e.g., consecutive) cell does.

In some aspects, the administration of HSCT subsequent to treatment, or initiation of treatment, with RAR cell therapy may be contraindicated. In some embodiments, subsequent HSCT may be considered within a specific period of time subsequent to initiation or termination of cell therapy, e.g., within about 1, about 2, about 3, about 4, about 5, about 6, about 7, about 8, about 9, about 10, about 11, or about 12 months after the administration or initiation of such therapy. In specific embodiments, patients having a cancer with one or more specified properties or attributes and that have received the cell dose or doses according to the method should not receive subsequent HSCT, should not receive subsequent HSCT unless other treatment options have been exhausted and/or may exhibit an increased risk of complication-induced death if receiving subsequent HSCT. In aspects of such embodiments, the one or more specified properties or attributes of the subject or disease are or include: morphologic disease, blasts greater than about 5%, about 20%, or about 50%, minimal disease, molecular disease; being above a certain specified age, such as an adult, at least 18, 20, 30, 40, 45, 50, 55, 60, 65, 70, 75, 80, 85, or 90 years of age; the presence of one or more comorbid diseases or conditions in the individual in addition to the disease or condition being treated, such as those that are known to present an increased risk of complication following stem cell transplant. For example, in some embodiments, if the subject has or is suspected of having one or more of the attributes, each individually or in any combination, subsequent HSCT is contraindicated or not recommended. In some embodiments, complication-induced death indicates death induced by a factor other than one directly attributed to the cell therapy, disease burden, and/or cancer (e.g. sepsis as a result of HSCT). In some embodiments, these patients may have achieved a complete remission of the cancer in response to the cell therapy, but may be at increased risk for non-cancer-attributable complications and/or death if administered an HSCT subsequent to cell therapy. In some aspects, the present methods involve the selection of a patient having a cancer exhibiting morphologic disease, or selecting a subject having minimal disease, comprising administering to the patient a dose of cells expressing a recombinant antigen receptor, and not administering a hematopoietic stem cell transplant (HSCT) to the subject subsequently to administering the antigen receptor-expressing cells. In some embodiments, the subject exhibits the minimal disease and/or is selected as having minimal disease. In some embodiments, at least at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission. In some embodiments, at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.

In some embodiments, the methods include administration of the RAR cells or a composition containing the cells to a subject, tissue, or cell, such as one having, at risk for, or suspected of having the disease, condition or disorder. In some embodiments, the RAR cells, populations, and/or compositions are administered to a subject having the particular disease or condition to be treated, e.g., via adoptive cell therapy, such as adoptive T cell therapy. In some embodiments, the RAR cells or compositions are administered to the subject, such as a subject having or at risk for the disease or condition, ameliorate one or more symptom of the disease or condition.

In some embodiments, the subject is naive for HSCT (i.e., HSCT-naive) and/or is naive for one or more other treatments for the disease or condition currently subject to treatment and/or for cancer therapy or chemotherapy or cytoreductive chemotherapy. In some embodiments, an HSCT-naïve subject (or subject naive for the other therapy) is one that has not ever received a hematopoietic stem cell transplant (or other therapy) and/or has not received it since diagnosis of or since acquiring this disease or condition currently subject to treatment. In some embodiments, the subject is maintained in such naive status for a certain period of time following the administration of the RAR cell dose or initiation of RAR cell therapy, such as for at least about 1, about 2, about 3, about 4, about 5, about 6, or about 12 months following the initiation of the administration of the first dose of RAR cells. In some embodiments, the subject has not been administered a HSCT prior to the initiation of the administering of the antigen receptor-expressing cells.

In some embodiments, the subject is one that has not been previously treated HSCT prior to the initiation of administration of the dose of cells expressing the recombinant antigen receptor, and/or has not been so treated within a particular time period or since diagnosis. In some aspects, the subject further does not receive an HSCT following the treatment or within a certain period of time following treatment and/or does not require such a treatment in order to achieve a particular survival or other effect.

In some embodiments, the subject has been previously treated with a hematopoietic stem cell transplantation (HSCT), e.g., allogenic HSCT, but does not receive a subsequent HSCT. In some embodiments, the subject has persistent or relapsed disease following HSCT.

In some embodiments, the subject has been treated with a therapeutic agent targeting the disease or condition, e.g. the tumor, prior to administration of the cells expressing the recombinant receptor, for example, to reduce or debulk disease but which does not completely eradicate the disease or condition, such as induction and/or consolidating chemotherapy. In some aspects, the subject is refractory or non-responsive to the other therapeutic agent. In some embodiments, the subject has persistent or relapsed disease, e.g., following treatment with another therapeutic intervention, including chemotherapy or radiation.

In some embodiments, the RAR cells are administered as part of a combination treatment, such as simultaneously with or sequentially with, in any order, another therapeutic intervention, such as an antibody or engineered cell or receptor or agent, such as a cytotoxic or therapeutic agent. The RAR cells in some embodiments are co-administered or concurrently administered with one or more additional therapeutic agents or in connection with another therapeutic intervention, either simultaneously or sequentially in any order. In some contexts, the RAR cells are co-administered with another therapy sufficiently close in time such that the RAR cell populations enhance the effect of one or more additional therapeutic agents, or vice versa. In some embodiments, the RAR cells are administered prior to the one or more additional therapeutic agents. In some embodiments, the RAR cells are administered after the one or more additional therapeutic agents. In some embodiments, the one or more additional agent includes a cytokine, such as IL-2, for example, to enhance persistence.

In some embodiments, the methods comprise administration of a chemotherapeutic agent, e.g., a conditioning chemotherapeutic agent, for example, to reduce tumor burden prior to the first or consecutive dose administrations.

In some embodiments, the administration effectively treats the subject despite the subject having become resistant to another therapy and/or despite the subject having not received another therapy or having not received another therapy other than another RAR cell dose or one or two rounds of chemotherapy. In some embodiments, subjects treated with the methods have an increased mean survival as compared to subjects treated with other methods.

The disease or condition that is treated is generally one with which expression of the target antigen is associated and/or involved in the etiology thereof, e.g. in which the antigen causes, exacerbates or otherwise is involved in such disease, condition, or disorder or is simply a marker of or is overexpressed or uniquely expressed on cells of the disease or disorder. Exemplary diseases and conditions can include diseases or conditions associated with malignancy or transformation of cells (e.g. cancer). Exemplary antigens, which include antigens associated with various diseases and conditions that can be treated, are described above. In particular embodiments, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors. In some embodiments, the subject has an infectious disease, such as infection with a virus or other pathogen, e.g., HIV, HCV, HBV, CMV, and parasitic disease.

In some embodiments, the disease or condition is a tumor, cancer, malignancy, neoplasm, or other proliferative disease or disorder. Such diseases include but are not limited to leukemia, lymphoma, e.g., chronic lymphocytic leukemia (CLL), acute-lymphoblastic leukemia (ALL), non-Hodgkin's lymphoma, acute myeloid leukemia, multiple myeloma, refractory follicular lymphoma, mantle cell lymphoma, indolent B cell lymphoma, B cell malignancies, cancers of the colon, lung, liver, breast, prostate, ovarian, skin, melanoma, bone, and brain cancer, ovarian cancer, epithelial cancers, renal cell carcinoma, pancreatic adenocarcinoma, Hodgkin's lymphoma, cervical carcinoma, colorectal cancer, glioblastoma, neuroblastoma, Ewing sarcoma, medulloblastoma, osteosarcoma, synovial sarcoma, and/or mesothelioma. In some embodiments, the subject has acute-lymphoblastic leukemia (ALL). In some embodiments, the subject has non-Hodgkin's lymphoma.

In some embodiments, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some embodiments, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some embodiments, the antigen associated with the disease or disorder is selected from the group consisting of orphan tyrosine kinase receptor ROR1, tEGFR, Her2, Ll-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, 0EPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, L1-cell adhesion molecule, MAGE-A1, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, CS-1, c-Met, GD-2, and MAGE A3, CE7, Wilms Tumor 1 (WT-1), a cyclin, such as cyclin A1 (CCNA1), and/or biotinylated molecules, and/or molecules expressed by HIV, HCV, HBV or other pathogens.

In some embodiments, the provided methods can enhance overall survival of subjects in which subjects exhibit a particular level of disease burden at the time of treatment such as morphologic or minimal disease.

Methods for administration of cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

In some embodiments, the cell therapy, e.g., adoptive cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, and following isolation and processing are administered to the same subject.

In some embodiments, the cell therapy, e.g., adoptive cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subj ect.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells. In some embodiments, it is administered by multiple bolus administrations of the cells, for example, over a period of no more than 3 days, or by continuous infusion administration of the cells.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some embodiments suitably administered to the subject at one time or over a series of treatments.

Once the cells are administered to the subject (e.g., human), the biological activity of the engineered cell populations in some aspects is measured by any of a number of known methods. Parameters to assess include specific binding of an engineered or natural T cell or other immune cell to antigen, in vivo, e.g., by imaging, or ex vivo, e.g., by ELISA or flow cytometry. In certain embodiments, the ability of the engineered cells to destroy target cells can be measured using any suitable method known in the art, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the cells also can be measured by assaying expression and/or secretion of certain cytokines, such as CD 107a, IFNγ, IL-2, and TNF. In some aspects the biological activity is measured by assessing clinical outcome, such as reduction in tumor burden or load. In some aspects, toxic outcomes, persistence and/or expansion of the cells, and/or presence or absence of a host immune response, are assessed.

In certain embodiments, engineered cells are modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known in the art. See, for instance, Wadwa et al., J. Drug Targeting 3: 1 1 1 (1995), and U.S. Patent 5,087,616.

### Recombinant Antigen Receptors Expressed by the Cells

In some embodiments, the cells for use in or administered in connection with the provided methods contain or are engineered to contain an engineered receptor, e.g., an engineered antigen receptor, such as a chimeric antigen receptor (CAR), or a T cell receptor (TCR). Also provided are populations of such cells, compositions containing such cells and/or enriched for such cells, such as in which cells of a certain type such as T cells or CD8⁺ or CD4⁺ cells are enriched or selected. Among the compositions are pharmaceutical compositions and formulations for administration, such as for adoptive cell therapy. Also provided are therapeutic methods for administering the cells and compositions to subjects, e.g., patients, in accord with the provided methods.

In some embodiments, the cells include one or more nucleic acids introduced via genetic engineering, and thereby express recombinant or genetically engineered products of such nucleic acids. In some embodiments, gene transfer is accomplished by first stimulating the cells, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

The cells generally express recombinant receptors, such as antigen receptors including functional non-TCR antigen receptors, e.g., chimeric antigen receptors (CARs), and other antigen-binding receptors such as transgenic T cell receptors (TCRs). Also among the receptors are other chimeric receptors.

### Chimeric Antigen Receptors (CARs)

In some embodiments of the provided methods and uses, chimeric receptors, such as a chimeric antigen receptors, contain one or more domains that combine a ligand-binding domain (e.g. antibody or antibody fragment) that provides specificity for a desired antigen (e.g., tumor antigen) with intracellular signaling domains. In some embodiments, the intracellular signaling domain is an activating intracellular domain portion, such as a T cell activating domain, providing a primary activation signal. In some embodiments, the intracellular signaling domain contains or additionally contains a costimulatory signaling domain to facilitate effector functions. In some embodiments, chimeric receptors when genetically engineered into immune cells can modulate T cell activity, and, in some cases, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

The chimeric receptors, such as CARs, generally include an extracellular antigen binding domain, such as a portion of an antibody molecule, generally a variable heavy (V_{H}) chain region and/or variable light (V_{L}) chain region of the antibody, e.g., an scFv antibody fragment.

In some embodiments, the antigen targeted by the receptor is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

Antigens targeted by the receptors in some embodiments include orphan tyrosine kinase receptor ROR1, tEGFR, Her2, Ll-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, 0EPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, L1-cell adhesion molecule, MAGE-A1, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, and MAGE A3, CE7, Wilms Tumor 1 (WT-1), a cyclin, such as cyclin A1 (CCNA1), and/or biotinylated molecules, and/or molecules expressed by HIV, HCV, HBV or other pathogens.

In some embodiments, the CAR binds a pathogen-specific antigen. In some embodiments, the CAR is specific for viral antigens (such as HIV, HCV, HBV, etc.), bacterial antigens, and/or parasitic antigens.

In some embodiments, the chimeric antigen receptor includes an extracellular portion containing an antibody or antibody fragment. In some aspects, the chimeric antigen receptor includes an extracellular portion containing the antibody or fragment and an intracellular signaling domain. In some embodiments, the antibody or fragment includes an scFv.

In some embodiments, the antibody portion of the recombinant receptor, e.g., CAR, further includes at least a portion of an immunoglobulin constant region, such as a hinge region, e.g., an IgG4 hinge region, and/or a CH1/CL and/or Fc region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, international patent application publication number WO2014031687, U.S. Patent No. 8,822,647 or published app. No. US2014/0271635.

In some embodiments, the antigen receptor comprises an intracellular domain linked directly or indirectly to the extracellular domain. In some embodiments, the chimeric antigen receptor includes a transmembrane domain linking the extracellular domain and the intracellular signaling domain. In some embodiments, the intracellular signaling domain comprises an ITAM. For example, in some aspects, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some embodiments, the chimeric receptor comprises a transmembrane domain linked or fused between the extracellular domain (e.g., scFv) and intracellular signaling domain . Thus, in some embodiments, the antigen-binding component (e.g., antibody) is linked to one or more transmembrane and intracellular signaling domains.

In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CDS, CD9, CD 16, CD22, CD33, CD37, CD64, CD80, CD86, CD 134, CD137, CD 154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s). In some aspects, the transmembrane domain contains a transmembrane portion of CD28. In some embodiments, the receptor further includes a portion of the extracellular region of a CD28, such as that in the chimeric receptors described in U.S. Patent No. 7,446,190. In some embodiments, for example, where the antigen is CD19, the chimeric receptor is one as described in U.S. Patent No. 7,446,190, such as one having a CD28-derived sequence as described therein and an anti-CD19 scFv, and in some aspects, further having a human CD3zeta intracellular portion. In some embodiments, it is a 1928z. Exemplary structure and sequences of 1928z can be found in international patent application publication number WO 2014134165, WO 2008121420, and U.S. Patent Number 7,446,190.

In some embodiments, the extracellular domain and transmembrane domain can be linked directly or indirectly. In some embodiments, the extracellular domain and transmembrane are linked by a spacer, such as any described herein. In some embodiments, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

The receptor, e.g., the CAR, generally includes at least one intracellular signaling component or components. In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some embodiments, cytoplasmic signaling molecule(s) in the CAR contain(s) a cytoplasmic signaling domain, portion thereof, or sequence derived from CD3 zeta.

In some embodiments, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some embodiments, cell signaling modules include CD3 transmembrane domain, CD3 intracellular signaling domains, and/or other CD transmembrane domains. In some embodiments, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some embodiments, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling domain of an antigen receptor component or costimulatory molecule is used in place of an intact immunostimulatory chain, for example, if it transduces the effector function signal. In some embodiments, the intracellular signaling domain or domains include the cytoplasmic sequences of the T cell receptor (TCR), and in some aspects also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

In some embodiments, the chimeric antigen receptor contains an intracellular domain of a T cell costimulatory molecule. In some embodiments, the CAR includes a signaling domain and/or transmembrane portion of a costimulatory receptor, such as CD28, 4-1BB, OX40, DAP10, and ICOS. In some aspects, the same CAR includes both the activating and costimulatory components. In some embodiments, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 41BB.

In some embodiments, the activating domain is included within one CAR, whereas the costimulatory component is provided by another CAR recognizing another antigen. In some embodiments, the CARs include activating or stimulatory CARs, costimulatory CARs, both expressed on the same cell (see WO2014/055668). In some aspects, the cells include one or more stimulatory or activating CAR and/or a costimulatory CAR. In some embodiments, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In certain embodiments, the intracellular signaling domain comprises a CD28 transmembrane and signaling domain linked to a CD3 (e.g., CD3-zeta) intracellular domain. In some embodiments, the intracellular signaling domain comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some embodiments, the antigen receptor further includes a marker and/or cells expressing the CAR or other antigen receptor further includes a surrogate marker, such as a cell surface marker, which may be used to confirm transduction or engineering of the cell to express the receptor. In some aspects, the marker includes all or part (e.g., truncated form) of CD34, a NGFR, or epidermal growth factor receptor, such as truncated version of such a cell surface receptor (e.g., tEGFR). In some embodiments, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (EGFRt) that is, optionally, linked to a linker sequence, such as a T2A cleavable linker sequence.

In some embodiments, the marker is a molecule, e.g., cell surface protein, not naturally found on T cells or not naturally found on the surface of T cells, or a portion thereof. In some embodiments, the molecule is a non-self molecule, e.g., non-self protein, i.e., one that is not recognized as "self' by the immune system of the host into which the cells will be adoptively transferred.

In some embodiments, the marker serves no therapeutic function and/or produces no effect other than to be used as a marker for genetic engineering, e.g., for selecting cells successfully engineered. In other embodiments, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

For example, in some embodiments, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer..

For example, in some embodiments, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some embodiments, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In some embodiments, nucleic acid molecules encoding such CAR constructs further includes a sequence encoding a T2A ribosomal skip element and/or a tEGFR sequence, e.g., downstream of the sequence encoding the CAR. In some embodiments, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374).

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some embodiments, the cells express a CAR that specifically binds to an antigen expressed by a cell or tissue of the disease or condition or associated with the disease or condition.

### TCRs

In some embodiments, the genetically engineered antigen receptors include recombinant T cell receptors (TCRs) and/or TCRs cloned from naturally occurring T cells. In some embodiments, a high-affinity T cell clone for a target antigen (e.g., a cancer antigen) is identified, isolated from a patient, and introduced into the cells. In some embodiments, the TCR clone for a target antigen has been generated in transgenic mice engineered with human immune system genes (e.g., the human leukocyte antigen system, or HLA). See, e.g., tumor antigens (see, e.g., Parkhurst et al. (2009) Clin Cancer Res. 15:169-180 and Cohen et al. (2005) J Immunol. 175:5799-5808. In some embodiments, phage display is used to isolate TCRs against a target antigen (see, e.g., Varela-Rohena et al. (2008) Nat Med. 14:1390-1395 and Li (2005) Nat Biotechnol. 23:349-354.

In some embodiments, after the T-cell clone is obtained, the TCR alpha and beta chains are isolated and cloned into a gene expression vector. In some embodiments, the TCR alpha and beta genes are linked via a picornavirus 2A ribosomal skip peptide so that both chains are coexpression. In some embodiments, genetic transfer of the TCR is accomplished via retroviral or lentiviral vectors, or via transposons (see, e.g., Baum et al. (2006) Molecular Therapy: The Journal of the American Society of Gene Therapy. 13:1050-1063; Frecha et al. (2010) Molecular Therapy: The Journal of the American Society of Gene Therapy. 18:1748-1757; an Hackett et al. (2010) Molecular Therapy: The Journal of the American Society of Gene Therapy. 18:674-683.

### Dosing

In some embodiments, the size or timing of the doses is determined as a function of the particular disease or condition in the subject. It is within the level of a skilled artisan to empirically determine the size or timing of the doses for a particular disease in view of the provided description.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose, provided in multiple individual compositions or infusions, over a specified period of time, which is no more than 3 days. Thus, in some contexts, the first or consecutive dose is a single or continuous administration of the specified number of cells, given or initiated at a single point in time. In some contexts, however, the first or consecutive dose is administered in multiple injections or infusions over a period of no more than three days, such as once a day for three days or for two days or by multiple infusions over a single day period.

Thus, in some aspects, the cells of the first dose are administered in a single pharmaceutical composition. In some embodiments, the cells of the consecutive dose are administered in a single pharmaceutical composition.

In some embodiments, the cells of the first dose are administered in a plurality of compositions, collectively containing the cells of the first dose. In some embodiments, the cells of the consecutive dose are administered in a plurality of compositions, collectively containing the cells of the consecutive dose. In some aspects, additional consecutive doses may be administered in a plurality of compositions over a period of no more than 3 days.

The term "split dose" refers to a dose that is split so that it is administered over more than one day. This type of dosing is encompassed by the present methods and is considered to be a single dose.

Thus, the dose of cells may be administered as a split dose. For example, in some embodiments, the dose may be administered to the subject over 2 days or over 3 days. Exemplary methods for split dosing include administering about 25% of the dose on the first day and administering the remaining about 75% of the dose on the second day. In other embodiments, about 33% of the first dose may be administered on the first day and the remaining about 67% administered on the second day. In some aspects, about 10% of the dose is administered on the first day, about 30% of the dose is administered on the second day, and about 60% of the dose is administered on the third day. In some embodiments, the split dose is not spread over more than 3 days.

In some embodiments, the dose of cells is generally large enough to be effective in reducing disease burden.

In some embodiments, the cells are administered at a desired dosage, which in some aspects includes a desired dose or number of cells or cell type(s) and/or a desired ratio of cell types. Thus, the dosage of cells in some embodiments is based on a total number of cells (or number per kg body weight) and a desired ratio of the individual populations or sub-types, such as the CD4⁺ to CD8⁺ ratio. In some embodiments, the dosage of cells is based on a desired total number (or number per kg of body weight) of cells in the individual populations or of individual cell types. In some embodiments, the dosage is based on a combination of such features, such as a desired number of total cells, desired ratio, and desired total number of cells in the individual populations.

In some embodiments, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, e.g., cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), e.g., within a certain tolerated difference or error of such a ratio.

In some embodiments, the cells are administered at or within a tolerated difference of a desired dose of one or more of the individual populations or sub-types of cells, such as a desired dose of CD4⁺ cells and/or a desired dose of CD8⁺ cells. In some aspects, the desired dose is a desired number of cells of the sub-type or population, or a desired number of such cells per unit of body weight of the subject to whom the cells are administered, e.g., cells/kg. In some aspects, the desired dose is at or above a minimum number of cells of the population or sub-type, or minimum number of cells of the population or sub-type per unit of body weight.

Thus, in some embodiments, the dosage is based on a desired fixed dose of total cells and a desired ratio, and/or based on a desired fixed dose of one or more, e.g., each, of the individual sub-types or sub-populations. Thus, in some embodiments, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In certain embodiments, the cells, or individual populations of sub-types of cells, are administered to the subject at a range of about one million to about 100 billion cells, such as, e.g., about 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges.

In some embodiments, the dose of total cells and/or dose of individual sub-populations of cells is within a range of between about 10⁴ and about 10⁹ cells/kilograms (kg) body weight, such as between about 10⁵ and about 10⁶ cells / kg body weight, for example, at about 1 × 10⁵ cells/kg, about 1.5 × 10⁵ cells/kg, about 2 × 10⁵ cells/kg, or about 1 × 10⁶ cells/kg body weight. For example, in some embodiments, the cells are administered at, or within a certain range of error of, between about 10⁴ and about 10⁹ T cells/kilograms (kg) body weight, such as between about 10⁵ and about 10⁶ T cells / kg body weight, for example, at about 1 × 10⁵ T cells/kg, about 1.5 × 10⁵ T cells/kg, about 2 × 10⁵ T cells/kg, or about 1 × 10⁶ T cells/kg body weight.

In some embodiments, the cells are administered at or within a certain range of error of between about 10⁴ and about 10⁹ CD4⁺ and/or CD8⁺ cells/kilograms (kg) body weight, such as between about 10⁵ and about 10⁶ CD4⁺ and/or CD8⁺ cells / kg body weight, for example, at about 1 × 10⁵ CD4⁺ and/or CD8⁺ cells/kg, about 1.5 × 10⁵ CD4⁺ and/or CD8⁺ cells/kg, about 2 × 10⁵ CD4⁺ and/or CD8⁺ cells/kg, or about 1 × 10⁶ CD4⁺ and/or CD8⁺ cells/kg body weight.

In some embodiments, the cells are administered at or within a certain range of error of, greater than, and/or at least about 1 × 10⁶, about 2.5 × 10⁶, about 5 × 10⁶, about 7.5 × 10⁶, or about 9 × 10⁶ CD4⁺ cells, and/or at least about 1 × 10⁶, about 2.5 × 10⁶, about 5 × 10⁶, about 7.5 × 10⁶, or about 9 × 10⁶ CD8+ cells, and/or at least about 1 × 10⁶, about 2.5 × 10⁶, about 5 × 10⁶, about 7.5 × 10⁶, or about 9 × 10⁶ T cells. In some embodiments, the cells are administered at or within a certain range of error of between about 10⁸ and about 10¹² or between about 10¹⁰ and 10¹¹ T cells, between about 10⁸ and about 10¹² or between about 10¹⁰ and about 10¹¹ CD4⁺ cells, and/or between about 10⁸ and 10¹² or between about 10¹⁰ and about 10¹¹ CD8⁺ cells.

In some embodiments, the cells are administered at or within a tolerated range of a desired output ratio of multiple cell populations or sub-types, such as CD4⁺ and CD8⁺ cells or sub-types. In some aspects, the desired ratio can be a specific ratio or can be a range of ratios. for example, in some embodiments, the desired ratio (e.g., ratio of CD4⁺ to CD8⁺ cells) is between about 1:5 and about 5:1 (or greater than about 1:5 and less than about 5:1), or between about 1:3 and about 3:1 (or greater than about 1:3 and less than about 3:1), such as between about 2:1 and about 1:5 (or greater than about 1:5 and less than about 2:1, such as at about 5:1, about 4.5:1, about 4:1, about 3.5:1, about 3:1, about 2.5:1, about 2:1, about 1.9:1, about 1.8:1, about 1.7:1, about 1.6:1, about 1.5:1, about 1.4:1, about 1.3:1, about 1.2:1, about 1.1:1, about 1:1, about 1:1.1, about 1:1.2, about 1:1.3, about 1:1.4, about 1:1.5, about 1:1.6, about 1:1.7, about 1:1.8, about 1:1.9, about 1:2, about 1:2.5, about 1:3, about 1:3.5, about 1:4, about 1:4.5, or about 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In some embodiments, for example, where the subject is a human, the dose includes fewer than or equal to about 1 × 10⁸ total recombinant receptor (e.g., CAR)-expressing cells, T cells, or peripheral blood mononuclear cells (PBMCs), e.g., in the range of about 1 × 10⁶ to about 1 × 10⁸ such cells, inclusive, such as no more than about 2 × 10⁶, about 5 × 10⁶, about 1 × 10⁷, about 5 × 10⁷, or about 1 × 10⁸ or total such cells, or the range between any two of the foregoing values. In some embodiments, the dose contains fewer than or equal to about 1 × 10⁸ total recombinant receptor (e.g., CAR)-expressing cells, T cells, or peripheral blood mononuclear cells (PBMCs) cells per m² of the subject, e.g., in the range of about 1 × 10⁶ to about 1 × 10⁸ such cells per m² of the subject, inclusive, such as no more than about 2 × 10⁶, about 5 × 10⁶, about 1 × 10⁷, about 5 × 10⁷, or about 1 × 10⁸ such cells per m² of the subject, or the range between any two of the foregoing values.

In some embodiments, the number of cells in the dose is between about 0.5 × 10⁶ cells/kg body weight of the subject and about 3 × 10⁶ cells/kg, between about 0.75 × 10⁶ cells/kg and about 2.5 × 10⁶ cells/kg or between about 1 × 10⁶ cells/kg and about 2 × 10⁶ cells/kg, each inclusive.

In particular embodiments, the dose contains a number of cells, number of recombinant receptor (e.g., CAR)-expressing cells, number of T cells, or number of peripheral blood mononuclear cells (PBMCs) in the range from about 10⁵ to about 10⁶ of such cells per kilogram body weight of the subject, inclusive, and/or a number of such cells that is no more than about 10⁵ or about 10⁶ such cells per kilogram body weight of the subject, inclusive. For example, in some embodiments, the dose includes less than or no more than or about 1 × 10⁵, about 2 × 10⁵, about 5 × 10⁵, or about 1 × 10⁶ of such cells per kilogram body weight of the subject. In some embodiments, the dose includes at about 1 × 10⁵, at about 2 × 10⁵, at about 5 × 10⁵, or at about 1 × 10⁶ of such cells per kilogram body weight of the subject, or a value within the range between any two of the foregoing values.

In particular embodiments, the numbers and/or concentrations of cells refer to the number of recombinant receptor (e.g., CAR)-expressing cells. In other embodiments, the numbers and/or concentrations of cells refer to the number or concentration of all cells, T cells, or peripheral blood mononuclear cells (PBMCs) administered.

In some aspects, the size of the dose is determined based on one or more criteria such as response of the subject to prior treatment, e.g. chemotherapy, disease burden in the subject, such as tumor load, bulk, size, or degree, extent, or type of metastasis, stage, and/or likelihood or incidence of the subject developing toxic outcomes, e.g., CRS, macrophage activation syndrome, tumor lysis syndrome, neurotoxicity, and/or a host immune response against the cells and/or recombinant receptors being administered.

In some aspects, the size of the dose is determined by the burden of the disease or condition in the subject. For example, in some aspects, the number of cells administered is determined based on the tumor burden that is present in the subject immediately prior to administration of the dose of cells. In some embodiments, the size of the dose is inversely correlated with disease burden. In some aspects, as in the context of a large disease burden, the subject is administered a low number of cells, for example less than or equal to or about 1 × 10⁶ cells per kilogram of body weight of the subject or less than or equal to or about 0.5 × 10⁶ cells/kg. In other embodiments, as in the context of a lower disease burden, the subject is administered a larger number of cells, such as more than about 1 × 10⁶ cells per kilogram body weight of the subject, for example more than about 2 × 10⁶, about 2.5 × 10⁶ or about 3 × 10⁶ cells/kg. In some embodiments, the number of cells, recombinant receptor (e.g., CAR)-expressing cells, T cells, or peripheral blood mononuclear cells (PBMCs) in the dose is greater than about 1 × 10⁶ such cells per kilogram body weight of the subject, e.g., about 2 × 10⁶, about 3 × 10⁶, about 5 × 10⁶, about 1 × 10⁷, about 5 × 10⁷, about 1 × 10⁸, about 1 × 10⁹, or about 1 × 10¹⁰ such cells per kilogram of body weight and/or about 1 × 10⁸, about 1 × 10⁹, about 1 × 10¹⁰ such cells per m² of the subject or total, or the range between any two of the foregoing values.

In some embodiments, a subject is assessed for disease burden prior to treatment, and, if the subject exhibits less than 5% bone marrow blast cells, the subject can be administered a dose of recombinant receptor-expressing (e.g., CAR-expressing, such as CAR-expressing T cells) of greater than about 1 × 10⁶ cells/kg, about 2 × 10⁶ cells/kg, about 5 × 10⁶ cells/kg or about 1 × 10⁷ cells/kg, or if the subject exhibits greater than or equal to about 5% bone marrow blast cells, the subject can be administered a dose of recombinant receptor-expressing (e.g., CAR-expressing, such as CAR-expressing T cells) of less than or equal to or about 1 × 10⁶ cell/kg or about 0.5 × 10⁶ cells/kg. In some embodiments, a subject is assessed for disease burden prior to treatment, and, if the subject exhibits less than about 10%(e.g., less than about 9%, less than about 8%, less than about 7%, less than about 6%, less than about 5%, less than about 4%, less than about 3%, less than about 2%, less than about 1%, less than about 0.5%) bone marrow blast cells, the subject can be administered a dose of recombinant receptor-expressing (e.g., CAR-expressing, such as CAR-expressing T cells) of greater than about 1 × 10⁶ cells/kg, about 2 × 10⁶ cells/kg, about 5 × 10⁶ cells/kg or about 1 × 10⁷ cells/kg, or if the subject exhibits greater than or equal to about 10% bone marrow blast cells, the subject can be administered a dose of recombinant receptor-expressing (e.g., CAR-expressing, such as CAR-expressing T cells) of less than or equal to or about 1 × 10⁶ cell/kg or about 0.5 × 10⁶ cells/kg. In some embodiments, a subject is assessed for disease burden prior to treatment, and, if the subject exhibits less than about 20% bone marrow blast cells, the subject can be administered a dose of recombinant receptor-expressing (e.g., CAR-expressing, such as CAR-expressing T cells) of greater than about 1 × 10⁶ cells/kg, about 2 × 10⁶ cells/kg, about 5 × 10⁶ cells/kg or about 1 × 10⁷ cells/kg, or if the subject exhibits greater than or equal to about 20% bone marrow blast cells, the subject can be administered a dose of recombinant receptor-expressing (e.g., CAR-expressing, such as CAR-expressing T cells) of less than or equal to or about 1 × 10⁶ cell/kg or about 0.5 × 10⁶ cells/kg. In some embodiments, the disease burden is assessed and a risk-adapted dose is selected prior to the first dose of recombinant receptor-expressing (e.g., CAR-expressing, such as CAR-expressing T cells). In some embodiments, the disease burden is assessed and a risk-adapted dose is selected prior to one or more consecutive doses of recombinant receptor-expressing (e.g., CAR-expressing, such as CAR-expressing T cells).

In reference to cell numbers, in some embodiments, such values refer to numbers of recombinant receptor-expressing (e.g. CAR-expressing) cells; in other embodiments, they refer to number of T cells or PBMCs or total cells administered.

In some embodiments, one or more further consecutive doses can be administered. In some embodiments, the number of cells administered in the consecutive dose is the same as or similar to the number of cells administered in the first dose in any of the embodiments herein. In some embodiments, the particular dosage regimen is chosen to reduce or minimize toxicity in the subject upon administration of the cells expressing the recombinant antigen receptor, such as described in PCT Patent Application, publication no. WO2016/064929.

In some cases, if desired or need in a particular disease or context, the provided methods involve a consecutive dose of cells administered at an increased number, and hence at a higher dose, than the first dose of cells. In some embodiments, methods of first administering a low dose of recombinant receptor-expressing (e.g., CAR-expressing) cells can reduce the disease burden in subjects, such as from morphological disease status to minimal disease, so that subsequent administration of a higher dose of recombinant receptor-expressing (e.g., CAR-expressing) cells in subjects is less likely to cause toxic outcomes in a majority of subjects treated.

In some embodiments, prior to administration of the consecutive dose, the subject is one that exhibits non-morphological disease, such as molecularly detectable disease and/or minimal disease. A subject is said to exhibit minimal disease if the subject exhibits less than about 5% blasts in the bone marrow. In certain embodiments, the subject with minimal disease exhibits molecularly detectable cancer. In some embodiments, the subject is selected based on disease or condition, such as leukemia or lymphoma, such as a B cell leukemia or lymphoma and/or acute lymphoblastic leukemia (ALL). In some embodiments, the subject exhibits the minimal disease and/or is selected as having minimal disease. In some embodiments, at least about 50%, at least about 60%, at least about 70%, at least about 75%, at least about 80%, at least about 85%, at least about 90%, or at least about 99% of subjects with the disease or condition having minimal disease immediately prior to administration, treated by the method, exhibit complete remission and/or a MRD-negative complete remission. In some embodiments, at least about 50%, at least about 60%, at least about 70%, at least about 75%, at least about 80%, at least about 85%, at least about 90%, or at least about 99% of subjects with the disease or condition having morphologic disease immediately prior to administration, treated by the method, exhibit complete remission and/or MRD-negative complete remission.

In some embodiments, a subject can be assessed for tumor burden after administration of the first dose and prior to administration of the consecutive dose to confirm that tumor burden has been reduced compared to tumor burden present prior to treatment with the first dose. In some embodiments, if assessment of the subject indicates tumor burden is reduced and/or that the subject exhibits non-morphological disease, for example molecularly detectable disease and/or minimal disease, the provided methods include administering a consecutive dose of recombinant receptor-expressing (e.g., CAR-expressing) cells that is higher than the first or initial dose. In some embodiments, if assessment of the subject indicates tumor burden is not reduced and/or the subject exhibits morphological disease, the provided methods include administering a consecutive dose of recombinant receptor-expressing (e.g., CAR-expressing) cells that is the same as or less than the first or initial dose.

In some embodiments, a consecutive dose of recombinant receptor-expressing (e.g., CAR-expressing) cells is administered to the subject at a time after administration of the first or initial dose of cells in which it is likely that tumor burden of the subject has been reduced by the first dose. In some embodiments, it is not necessary that the tumor burden actually be reduced in all subjects prior to administration of the consecutive dose, but that tumor burden is reduced on average in subjects treated, such as based on clinical data, in which a majority of subjects treated with such a first dose exhibit a reduced tumor burden, such as at least about 50%, about 60%, about 70%, about 80%, about 90%, about 95% or more of subjects treated with the first or initial dose exhibit a reduced tumor burden. Generally at a point in time after disease burden has been reduced by the first dose or is likely to have been reduced by the first dose, a consecutive dose is administered to the subject, thereby further reducing and/or eliminating disease or a symptom or outcome thereof or preventing expansion or progression thereof. The context of reduced disease burden at the time of the consecutive administration in some aspects reduces the likelihood of exhaustion of the transferred cells, thereby improving efficacy. The consecutive dose may be the same, lower, or a higher dose as compared with the first dose. In some embodiments, multiple consecutive doses are administered after a first dose.

In some embodiments, the consecutive dose of recombinant receptor-expressing (e.g., CAR-expressing) cells is administered at a dose that is higher than the first dose so that an increased number of recombinant receptor-expressing (e.g., CAR-expressing) cells is administered to the subject by the consecutive dose. In some embodiments, a higher dose of recombinant receptor-expressing (e.g., CAR-expressing) cells is one that can promote an increased response or efficacy, such as improved or greater reduction in tumor burden and/or an improved or greater overall survival time of the subject compared to that achieved by administration of a lower dose or number of cells. In some embodiments, because administration of the first dose of cells can reduce tumor burden in the subject, administration of the consecutive dose at a higher number of cells can avoid or minimize CRS and/or neurotoxicity in the subject after administration of the consecutive dose that can otherwise occur in subjects with morphological disease.

In some aspects, the consecutive dose is larger than the first dose. For example, in some embodiments, the consecutive dose contains more than about 1 × 10⁶ cells, recombinant receptor (e.g. CAR)-expressing cells, T cells, and/or PBMCs per kilogram body weight of the subject, such as about or at least about 2 × 10⁶, about 3 × 10⁶, about 5 × 10⁶, about 1 × 10⁷, about 1 × 10⁸, or about 1 × 10⁹ such cells per kilogram body weight of the subject. In some embodiments, the number of cells in the consecutive dose is between about 2 × 10⁶ cells/kg body weight of the subject and about 6 × 10⁶ cells/kg, between about 2.5 × 10⁶ cells/kg and about 5.0 × 10⁶ cells/kg, or between about 3.0 × 10⁶ cells/kg and about 4.0 × 10⁶ cells/kg, each inclusive. In some embodiments, the amount or size of the consecutive dose is sufficient to reduce disease burden or an indicator thereof, and/or one or more symptoms of the disease or condition. In some embodiments, the dose is of a size effective to improve survival of the subject, for example, to induce survival, relapse-free survival, or event-free survival of the subject for at least 6 months, or at least about 1, about 2, about 3, about 4, or about 5 years. In some embodiments, the number of cells, recombinant receptor (e.g. CAR)-expressing cells, T cells, and/or PBMCs administered and/or number of such cells administered per body weight of the subject in the consecutive dose is at least 2-fold, 5-fold, 10-fold, 50-fold, or 100-fold or more greater than the number administered in the first dose. In some embodiments, disease burden, tumor size, tumor volume, tumor mass, and/or tumor load or bulk is reduced following the consecutive dose by at least about 50%, about 60%, about 70%, about 80%, or about 90% or more compared to that immediately prior to the administration of the first dose or of the consecutive dose.

In other embodiments, the number of cells administered in the consecutive dose is lower than the number of cells administered in the first dose.

In some embodiments, multiple consecutive doses are administered following the first dose, such that an additional dose or doses are administered following administration of the consecutive dose. In some aspects, the number of cells administered to the subject in the additional dose or doses (i.e., the third, fourth, fifth, and so forth) is the same as or similar to the first dose and/or consecutive dose. In some embodiments, the additional dose or doses are larger than prior doses.

In some embodiments, the timing of the consecutive dose(s) in relation to the first and/or one another is designed to reduce the risk of unwanted toxic outcomes and promote maximum efficacy. In some embodiments, the consecutive dose, such as the same, lower or higher consecutive dose, is administered at a time at which disease burden remains reduced in the subject or reduced in subjects on average, such as based on clinical data, but at which the risk of CRS and/or neurotoxicity remain low. In some embodiments, a consecutive dose is generally given at a point in time relative to the first or previous dose at which the risk of a toxic outcome or symptom or biochemical indicator thereof-such as CRS or neurotoxicity, macrophage activation syndrome, or tumor lysis syndrome-is at or below an acceptable level. For example, the consecutive dose may be administered after a toxic outcome has peaked and is declining or has declined below an acceptable level following the initial dose. Thus, in some embodiments, the consecutive dose is administered at least about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, or about 27 days following the initiation of the first or prior dose, or greater than about 14 or about 15 days or about 21 days following the initiation of the first or prior dose. In some embodiments, the appropriate timing is determined by monitoring and/or assessing the presence of one or more symptoms or outcomes associated with the toxic event and delivering the consecutive dose after determining that the symptom or outcome is at or below an acceptable level.

### Reduction in Disease Burden, Efficacy and Survival

The administration in accord with the provided methods generally reduces or prevents the expansion or burden of the disease or condition in the subject. For example, where the disease or condition is a tumor, the methods generally reduce tumor size, bulk, metastasis, percentage of blasts in the bone marrow or molecularly detectable cancer and/or improve prognosis or survival or other symptom associated with tumor burden.

Disease burden can encompass a total number of cells of the disease in the subject or in an organ, tissue, or bodily fluid of the subject, such as the organ or tissue of the tumor or another location, e.g., which would indicate metastasis. For example, tumor cells may be detected and/or quantified in the blood or bone marrow in the context of certain hematological malignancies. Disease burden can include, in some embodiments, the mass of a tumor, the number or extent of metastases and/or the percentage of blast cells present in the bone marrow.

In some embodiments, a subject has leukemia. The extent of disease burden can be determined by assessment of residual leukemia in blood or bone marrow. In some embodiments, a subject exhibits morphologic disease if there are greater than or equal to 5% blasts in the bone marrow, for example, as detected by light microscopy, such as greater than or equal to about 10% blasts in the bone marrow, greater than or equal to about 20% blasts in the bone marrow, greater than or equal to about 30% blasts in the bone marrow, greater than or equal to about 40% blasts in the bone marrow or greater than or equal to about 50% blasts in the bone marrow. In some embodiments, a subject exhibits complete or clinical remission if there are less than about 5% blasts in the bone marrow.

In some embodiments, a subject may exhibit complete remission, but a small proportion of morphologically undetectable (by light microscopy techniques) residual leukemic cells are present. A subject is said to exhibit minimum residual disease (MRD) if the subject exhibits less than 5% blasts in the bone marrow and exhibits molecularly detectable cancer. In some embodiments, molecularly detectable cancer can be assessed using any of a variety of molecular techniques that permit sensitive detection of a small number of cells. In some aspects, such techniques include PCR assays, which can determine unique Ig/T-cell receptor gene rearrangements or fusion transcripts produced by chromosome translocations. In some embodiments, flow cytometry can be used to identify cancer cell based on leukemia-specific immunophenotypes. In some embodiments, molecular detection of cancer can detect as few as 1 leukemia cell in 100,000 normal cells. In some embodiments, a subject exhibits MRD that is molecularly detectable if at least or greater than 1 leukemia cell in 100,000 cells is detected, such as by PCR or flow cytometry. In some embodiments, the disease burden of a subject is molecularly undetectable or MRD, such that, in some cases, no leukemia cells are able to be detected in the subject using PCR or flow cytometry techniques.

In some aspects, the disease or condition persists following administration of the first dose and/or administration of the first dose is not sufficient to eradicate the disease or condition in the subject.

In some aspects, administration of the consecutive dose reduces disease burden as compared to disease burden at a time immediately prior to the first dose, or at a time immediately prior to the consecutive dose. In some aspects, for example in the context of relapse, administration of the consecutive dose effects a reduction in disease burden as compared to the peak level of disease burden following administration of the first dose.

In some embodiments, the method reduces the burden of the disease or condition, e.g., number of tumor cells, size of tumor, duration of patient survival or event-free survival, to a greater degree and/or for a greater period of time as compared to the reduction that would be observed with a comparable method using an alternative dosing regimen, such as one in which the subject receives a prior and/or subsequent HSCT and/or other therapy that generally carries a risk of harmful side effects for example in certain patient populations.

In some embodiments, the burden of a disease or condition in the subject is detected, assessed, or measured. Disease burden may be detected in some aspects by detecting the total number of disease or disease-associated cells, e.g., tumor cells, in the subject, or in an organ, tissue, or bodily fluid of the subject, such as blood or serum. In some embodiments, disease burden, e.g. tumor burden, is assessed by measuring the mass of a solid tumor and/or the number or extent of metastases. In some aspects, survival of the subject, survival within a certain time period, extent of survival, presence or duration of event-free or symptom-free survival, or relapse-free survival, is assessed. In some embodiments, any symptom of the disease or condition is assessed. In some embodiments, the measure of disease or condition burden is specified.

In some embodiments, the event-free survival rate or overall survival rate of the subject is improved by the methods, as compared with other methods. For example, in some embodiments, event-free survival rate or probability for subjects treated by the methods at 6 months following the first dose is greater than about 40%, greater than about 50%, greater than about 60%, greater than about 70%, greater than about 80%, greater than about 90%, or greater than about 95%. In some aspects, overall survival rate is greater than about 40%, greater than about 50%, greater than about 60%, greater than about 70%, greater than about 80%, greater than about 90%, or greater than about 95%. In some embodiments, the subject treated with the methods exhibits event-free survival, relapse-free survival, or survival to at least about 6 months, at least about 1 year, about 2 years, about 3 years, about 4 years, about 5 years, about 6 years, about 7 years, about 8 years, about 9 years, or about 10 years. In some embodiments, the time to progression is improved, such as a time to progression of greater than about 6 months, at least about 1 year, about 2 years, about 3 years, about 4 years, about 5 years, about 6 years, about 7 years, about 8 years, about 9 years, or about 10 years.

In some embodiments, following treatment by the method, the probability of relapse is reduced as compared to other methods. For example, in some embodiments, the probability of relapse at 6 months following the first dose is less than about 80%, less than about 70%, less than about 60%, less than about 50%, less than about 40%, less than about 30%, less than about 20%, or less than about 10%.

### Genetically Engineered Cells and Methods of Producing Cells

In some embodiments, the provided methods involve administering to a subject having a disease or condition cells expressing a recombinant antigen receptor. Various methods for the introduction of genetically engineered components, e.g., recombinant receptors, e.g., CARs or TCRs, are well known and may be used with the provided methods and compositions. Exemplary methods include those for transfer of nucleic acids encoding the receptors, including via viral, e.g., retroviral or lentiviral, transduction, transposons, and electroporation.

Among the cells expressing the receptors and administered by the provided methods are engineered cells. The genetic engineering generally involves introduction of a nucleic acid encoding the recombinant or engineered component into a composition containing the cells, such as by retroviral transduction, transfection, or transformation.

### Vectors and Methods for Genetic Engineering

In some embodiments, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some embodiments, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some embodiments, the cells, e.g., T cells, may be transfected either during or after expansion e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the CD3/CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

### Cells and Preparation of Cells for Genetic Engineering

In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some embodiments, the cells are derived from the blood, bone marrow, lymph, or lymphoid organs, are cells of the immune system, such as cells of the innate or adaptive immunity, e.g., myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some embodiments, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, and re-introducing them into the same subject, before or after cryopreservation.

Among the sub-types and subpopulations of T cells and/or of CD4+ and/or of CD8+ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some embodiments, the cells are natural killer (NK) cells. In some embodiments, the cells are monocytes or granulocytes, e.g., myeloid cells, macrophages, neutrophils, dendritic cells, mast cells, eosinophils, and/or basophils.

In some embodiments, the cells include one or more nucleic acids introduced via genetic engineering, and thereby express recombinant or genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

In some embodiments, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some embodiments, the cells are derived from cell lines, e.g., T cell lines. The cells in some embodiments are obtained from a xenogeneic source, for example, from mouse, rat, non-human primate, and pig.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca⁺⁺/Mg⁺⁺ free PBS. In certain embodiments, components of a blood cell sample are removed and the cells directly resuspended in culture media.

In some embodiments, the methods include density-based cell separation methods, such as the preparation of white blood cells from peripheral blood by lysing the red blood cells and centrifugation through a Percoll or Ficoll gradient.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using CD3/CD28 conjugated magnetic beads (e.g., DYNABEADS^{®} M-450 CD3/CD28 T Cell Expander).

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. *See* Terakuraet al. (2012) Blood. 1:72-82; Wang et al. (2012) J Immunother. 35(9):689-701. In some embodiments, combining T_{CM}-enriched CD8⁺ T cells and CD4⁺T cells further enhances efficacy.

In embodiments, memory T cells are present in both CD62L⁺ and CD62L⁻subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some embodiments, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or sub-population, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some embodiments, naive CD4⁺ T lymphocytes are CD45RO⁻, CD45RA⁺, CD62L⁺, CD4⁺ T cells. In some embodiments, central memory CD4⁺ cells are CD62L⁺ and CD45RO⁺. In some embodiments, effector CD4⁺ cells are CD62L⁻ and CD45RO⁻.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher ⓒ Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidincoated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some embodiments, the magnetizable particles are biodegradable.

In some embodiments, the affinity-based selection is via magnetic-activated cell sorting (MACS) (Miltenyi Biotech, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1.

In some embodiments, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotic), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakuraet al. (2012) Blood. 1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flowcytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some embodiments, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR component and/or costimulatory receptor, e.g., anti-CD3, anti-CD28, for example, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some embodiments, the stimulating agents include IL-2 and/or IL-15, for example, an IL-2 concentration of at least about 10 units/mL.

In some aspects, incubation is carried out in accordance with techniques such as those described in US Patent No. 6,040,1 77 to Riddell et al., Klebanoff et al.(2012) J Immunother. 35(9): 651-660, Terakuraet al. (2012) Blood. 1:72-82, and/or Wang et al. (2012) J Immunother. 35(9):689-701.

In some embodiments, the T cells are expanded by adding to a cultureinitiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gammairradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells.

In some embodiments, the stimulating conditions include temperature suitable for the growth of human T lymphocytes, for example, at least about 25 degrees Celsius, generally at least about 30 degrees, and generally at about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In embodiments, antigen-specific T cells, such as antigen-specific CD4+ and/or CD8+ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.

### Compositions and Formulations

In some embodiments, the dose of cells comprising cells engineered with a recombinant antigen receptor, e.g. CAR or TCR, is provided as a composition or formulation, such as a pharmaceutical composition or formulation. Such compositions can be used in accord with the provided methods, such as in the prevention or treatment of diseases, conditions, and disorders, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some embodiments, the agents or cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

Active ingredients may be entrapped in microcapsules, in colloidal drug delivery systems (for example, liposomes, albumin microspheres, microemulsions, nanoparticles and nanocapsules) or in macroemulsions. In certain embodiments, the pharmaceutical composition is formulated as an inclusion complex, such as cyclodextrin inclusion complex, or as a liposome. Liposomes can serve to target the agent or host cells (e.g., T-cells or NK cells) to a particular tissue. Many methods are available for preparing liposomes, such as those described in, for example, Szoka et al., Ann. Rev. Biophys. Bioeng., 9: 467 (1980), and U.S. Patents 4,235,871, 4,501,728, 4,837,028, and 5,019,369.

The pharmaceutical composition in some aspects can employ time-released, delayed release, and sustained release delivery systems such that the delivery of the composition occurs prior to, and with sufficient time to cause, sensitization of the site to be treated. Many types of release delivery systems are available and known. Such systems can avoid repeated administrations of the composition, thereby increasing convenience to the subject and the physician.

The pharmaceutical composition in some embodiments contains agents or cells in amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The agents or cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells or agent. In some embodiments, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some embodiments suitably administered to the subject at one time or over a series of treatments.

The cells or agents may be administered using standard administration techniques, formulations, and/or devices. Provided are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some embodiments, the agent or cell populations are administered parenterally. The term "parenteral," as used herein, includes intravenous, intramuscular, subcutaneous, rectal, vaginal, and intraperitoneal administration. In some embodiments, the agent or cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyoi (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

### Kits

Also provided are articles of manufacture, such as kits, which contain one or more cell doses according to any of the provided embodiments, such as in any of the formulations or compositions described (which may be present in one or more containers, such as bags or vials, with cells in sufficient numbers or concentration for administration of an individual dose or a portion thereof), and instructions and/or packaging materials or literature with instructions for the administration in accordance with a method as provided herein. In some aspects, the instructions indicate the caution or contraindication of any of the embodiments described.

### Definitions

The terms "polypeptide" and "protein" are used interchangeably to refer to a polymer of amino acid residues, and are not limited to a minimum length. Polypeptides, including the provided receptors and other polypeptides, e.g., linkers or peptides, may include amino acid residues including natural and/or non-natural amino acid residues. The terms also include post-expression modifications of the polypeptide, for example, glycosylation, sialylation, acetylation, and phosphorylation. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a "subject" is a mammal, such as a human or other animal, and typically is human. In some embodiments, the subject, e.g., patient, to whom the agent or agents, cells, cell populations, or compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

The term "about" as used herein refers to the usual error range for the respective value readily known to the skilled person in this technical field. Reference to "about" a value or parameter herein includes (and describes) embodiments that are directed to that value or parameter per se. For example, "about" can mean within 3 or more than 3 standard deviations, per the practice in the art. In addition, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As used herein, the singular forms "a," "an," and "the" include plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, non-aqueous or any combination thereof.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at even near 100 % in the enriched composition.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control or fluorescence minus one (FMO) gating control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control or fluorescence minus one (FMO) gating control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### EXAMPLES

The following examples are included for illustrative purposes only and are not intended to limit the scope of the invention.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### Example 1: Administration of CD19-Targeting CAR Modified T Cells in Adult Patients with Relapsed, Refractory B-Cell ALL

Adult patients with relapsed, refractory CD19⁺ B-cell acute lymphoblastic leukemia (B-ALL) were treated with anti-CD 19 chimeric antigen receptor (CAR)-expressing T cells. Briefly, leukapheresis was performed and T cells enriched from peripheral blood mononuclear cell (PBMC) samples from patients. These cells were transduced with a retroviral vector encoding a CD19 CAR expression cassette (19-28z; including an anti-CD 19 scFv, extracellular, transmembrane, and cytoplasmic portions of human CD28 and a CD3zeta signaling domain). Patients selected for treatment were at least 18 years of age and suffering from relapsed refractory CD19⁺ B-ALL (relapse after allogeneic hematopoietic stem cell transplantation (HSCT) and/or isolated extramedullary disease was permitted). Individuals were excluded if they suffered from any active central nervous system (CNS) disease, exhibited active Graft-versus-host disease requiring immunosuppressants, or significant heart disease (e.g., patients suffering from myocardial infarction within six months or having ejection fraction higher than 40%). Conditioning chemotherapy was administered two days prior to infusion with CAR-T cells (which were generally administered in at least one dose of 1 × 10⁶ or 3 × 10⁶ CAR-expressing cells per kg). Disease was assessed at day 28-35 post-infusion. In some cases, subjects received a second dose at approximately day 14 post-infusion.

Fifty (50) of fifty-one (51) patients treated were evaluable for response assessment with more than one month follow up (all 51 were evaluable for toxicity). Disease burden of each patient was assessed prior to T cell infusion. Thirty-one (31) patients morphologic disease (at least 5 % blasts in the bone marrow), whereas twenty (20) patients minimal disease (less than 5 % blasts in the bone marrow). Among the 50 patient evaluable for response assessment with more than one month follow up, 29 (58%) received follow up assessment for 6 months or more, and 17 (34%) received follow up assessment for one year or more. Baseline patient characteristics for these subjects are shown in Table 1.

**Table 1**

| **Characteristic** | **Morphologic Disease N=31 (%)** | **Minimal Disease N=20 (%)** |
|---|---|---|
| Age at infusion (years) | | |
| 18-29 | 8 (26) | 4(20) |
| 30-59 | 18 (58) | 10 (50) |
| ≥60 | 5(16) | 6(30) |
| Median (range) | 40(24-73) | 53 (22-74) |
| Prior Lines of Therapy | | |
| 2 | 8 (26) | 12 (60) |
| 3 | 8 (26) | 5 (25) |
| ≥4 | 15 (48) | 3 (15) |
| Median (range) | 3 (2 - 7) | 2 (2 - 4) |
| Prior Blinatumomab | 11 (36) | 1 (5) |
| Prior allogeneic HSCT | | |
| Yes | 13 (42) | 5 (25) |
| No | 18 (58) | 15 (75) |
| Philadelphia Chromosome + | 7(23) | 8 (40) |

Almost fifty percent (48%) of the morphologic disease cohort had had four (4) or more prior lines of therapy; 36% had received prior treatment with blinatumomab (a CD19-targeting therapy).

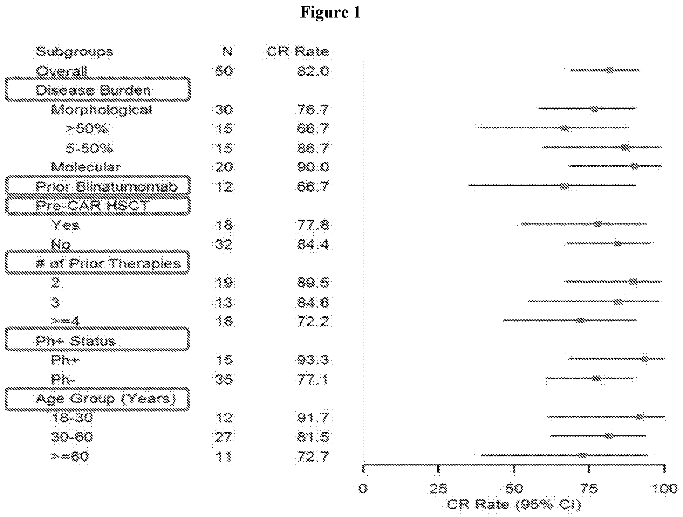

Complete remission (CR) rates, minimal residual disease (MRD)-negative (MRD-) rates and mean time to CR for the evaluable patients are shown in Table 2 (including for individual cohorts by baseline disease burden (Morphologic Disease (30) and Minimal Disease (20)). CR rates by various other subgroups are shown in Figure 1.

**Table 2**

| | **Morphologic Disease N=30 (%)** | **Minimal Disease N=20 (%)** |
|---|---|---|
| CR Rate | 23 (77%) [58 - 90] | 18 (90%) [68 - 99] |
| MRD negative CR Rate^{∗} | 19/21 (90%) [70 - 99] | 14/18 (78%) [52 - 94] |
| Time to CR, Mean (SD) | 20 days (9) | 25 days (9) |

| | | |
|---|---|---|
| ^{∗}Minimal residual disease (MRD) assessment was not available in 2 patients. | | |

Following the 19-28z CAR T cell infusion treatment, 16 of the 41 (39%) patients who achieved CR proceeded to allogeneic HSCT after achieving CR. Nine (9) (39%) of these 16 patients had morphologic disease at baseline and 7 had minimal disease at baseline. Among the 16 patients who proceeded to allogeneic HSCT, 14 had received no prior HSCT and 2 had received prior HSCT.

Among the 33 MRD-negative CR patients, 15 (45%) relapsed, whereas 9 (21 %) remained disease-free for more than a year. The disease in four (4) (27%) of the patients who relapsed was determined to be CD19-negative or CD19-undetectable.

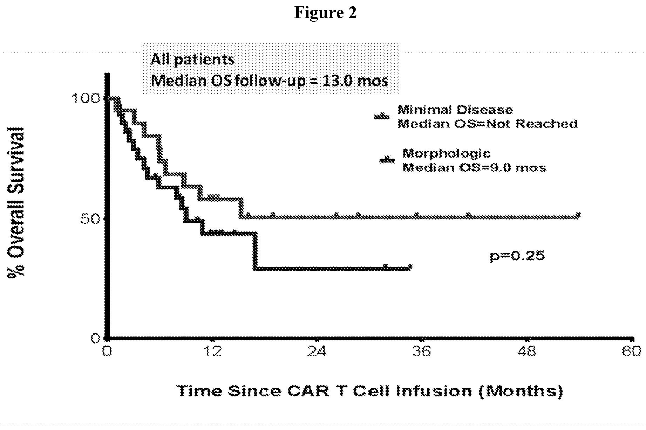

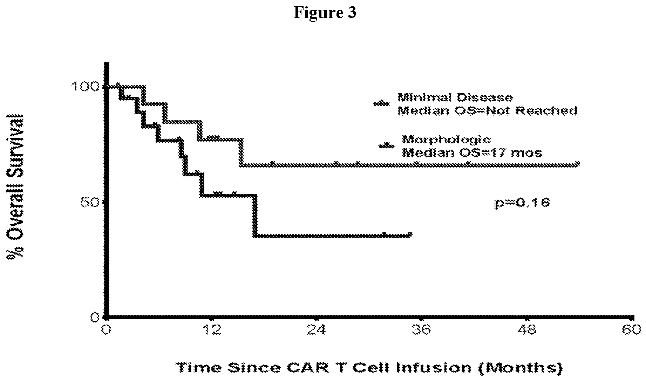

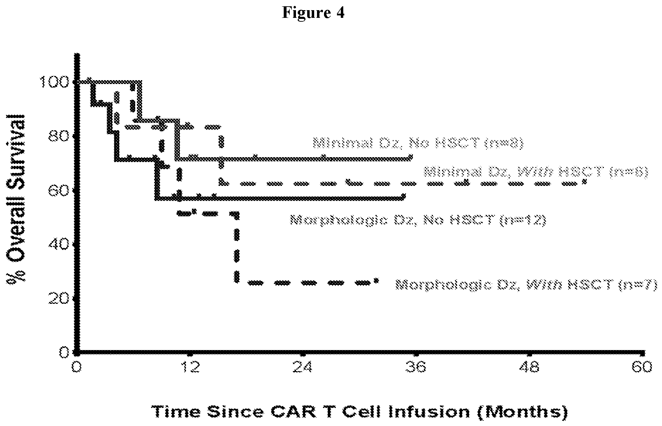

Overall survival of all patients grouped by baseline disease burden is reflected in Figure 2. Overall survival of MRD-CR patients grouped by baseline disease burden is reflected in Figure 3. Overall survival of patients grouped by baseline disease burden and post CAR-T HSCT is shown in Figure 4.

The CAR-engineered T cells were measured in peripheral blood (PB) and bone marrow (BM) by qPCR and flow cytometry. Maximum T cell expansion was detected at between 7 to 14 days after infusion, and correlated with occurrence of cytokine release syndrome (CRS). T cells persisted out to between 1 to 6 months following T cell infusion, as measured by qPCR.

Adverse events observed included CRS (e.g., fever, hypotension, and respiratory insufficiency), neurological changes (e.g., delirium, global encephalopathy, aphasia, seizure and seizure-like activities). In a majority of patients, neurological symptoms were reversible; neurotoxicity can occur independently of CRS. The grading system used in this example for CRS are summarized in Table 3. Other grading systems are known and may be used, such as a system as described by Lee et al, Blood. 2014;124(2):188-95, and/or as summarized in Table 3A. CRS and neurological toxicities observed in the patients grouped by baseline disease burden are shown in Table 4. Use of tocilizumab and/or steroids to treat toxicity in this study had no impact on relapse-free survival (RFS).

**Table 3**

| **Grade** | **Definitions** |
|---|---|
| Grade 1 | Mild symptoms, Fever, hypotension, nausea, myalgia, etc. requiring observation or symptomatic management only (e.g. antipyretics, IV fluids, antiemetics, pain medications, etc.) |
| Grade 2 | Moderate symptoms Hypotension requiring any vasopressors < 24 hours, or Hypoxia or dyspnea requiring supplemental oxygen ≤40% (i.e. <6L NC) |
| Grade 3 | Severe symptoms Hypotension requiring any vasopressors for 24-72 hours, or Hypoxia or dyspnea requiring supplemental oxygen >40% |
| Grade 4 | Life-threatening symptoms Hypotension requiring continuous vasopressors >72 hours, or Hypoxia or dyspnea requiring mechanical ventilation |
| Grade 5 | Death |

| | |
|---|---|
| Severe CRS = Grade 3 or 4 CRS | |

**Table 3A**

| **Exemplary Grading Criteria for CRS** | |
|---|---|
| **Grade** | **Description of Symptoms** |
| 1 | Not life-threatening, require only symptomatic treatment such as antipyretics and anti-emetics (e.g., fever, nausea, fatigue, headache, myalgias, malaise) |
| Mild | |
| 2 | Require and respond to moderate intervention: |
| Moderate | □ Oxygen requirement < 40%, or |
| | □ Hypotension responsive to fluids or low dose of a single vasopressor, or |
| | □ Grade 2 organ toxicity (by CTCAE v4.0) |
| 3 | Require and respond to aggressive intervention: |
| Severe | □ Oxygen requirement ≥ 40%, or |
| | □ Hypotension requiring high dose of a single vasopressor (e.g., norepinephrine ≥ 20 µg/kg/min, dopamine ≥ 10 µg/kg/min, phenylephrine ≥ 200 µg/kg/min, or epinephrine ≥ 10 µg/kg/min), or |
| | □ Hypotension requiring multiple vasopressors (e.g., vasopressin + one of the above agents, or combination vasopressors equivalent to ≥ 20 µg/kg/min norepinephrine), or |
| | □ Grade 3 organ toxicity or Grade 4 transaminitis (by CTCAE v4.0) |
| 4 | Life-threatening: |
| Life-threatening | □ Requirement for ventilator support, or |
| | □ Grade 4 organ toxicity (excluding transaminitis) |
| 5 | Death |
| Fatal | |

**Table 4**

| | **Morphologic Disease (N=31)** | **Minimal Disease (N=20)** |
|---|---|---|
| Severe CRS | 13 (42%) | 1(5%) |
| Grade 3/4 Neuro Toxicities | 11(35%) | 4 (20%) |
| Severe CRS & Gr 3/4 Neuro Tox | 7 (23%) | 1(5%) |
| Severe CRS or Gr 3/4 Neuro Tox | 17 (55%) | 4 (20%) |
| Grade 5 Toxicity | 4 (13%)^{∗∗} | |
| Management | | |
| Treated with Tocilizumab | 12 (39%) | 0 (0%) |
| Treated with Steroids | 11 (35%) | 1 (5%) |
| Treated with Toci & Steroids | 10 (32%) | 0 (0%) |

| | | |
|---|---|---|
| ^{∗∗}All patients received a higher dose (3×10⁶ CAR T cells/kg): 2 patients with sepsis/multi-organ failure; 1 patient had seizure, but unknown cause of death | | |

In this study among the patients assessed, CR rates of 77-90% and MRD-negative CR rates of 68-70% were observed. Durable responses and survival rates were observed in a subset of patients with no subsequent allogeneic HSCT in both morphologic and minimal disease cohorts. Patients with minimal disease at baseline exhibited no severe CRS and were observed to have lower rates of neurological toxicities as compared to the morphologic baseline disease burden cohort.

## Claims

1. A dose of cells expressing a recombinant antigen receptor for use in treating a subject having a disease or a condition, wherein the subject does not receive a hematopoietic stem cell transplant (HSCT) subsequent to the administration of the antigen receptor-expressing cells, optionally wherein the subject dose not receive an HSCT within about 1 month, about 2 months, about 3 months, about 4 months, about 5 months, about 6 months, about 7 months, about 8 months, about 9 months, about 10 months, about 11 months, or about 1 year subsequent to the initiation of the administration of the antigen receptor-expressing cells.

2. The cells for use of claim 1, wherein the subject:
(a) has not received an HSCT (i) prior to the administration the antigen receptor-expressing cells, and/or (ii) since diagnosis of the disease or condition; and/or
(b) does not receive an HSCT contemporaneous with the administration of the antigen receptor-expressing cells; and/or
(c) does not receive an HSCT in the absence of relapse of the subject, optionally wherein the subject does not receive an HSCT following a clinical remission; and/or
(d) is an adult, optionally the subject is at least about 18, 20, 30, 40, 50, 55, 60, 65, 70, 75, 80, or 85 years of age; and/or
(e) has not received a therapy for the disease or condition (i) prior to the administration the antigen receptor-expressing cells, and/or (ii) since diagnosis of the disease or condition, optionally wherein the therapy does not comprise induction chemotherapy, consolidating chemotherapy, and/or a prior administration of the antigen receptor-expressing cells; and/or
(f) exhibits morphologic disease or a minimum disease prior to the initial administration of the antigen receptor-expressing cells, optionally wherein the morphologic disease comprises greater than about 5%, greater than about 20%, or greater than about 50% blast cells in the bone marrow and/or the minimal disease comprises less than about 5% blasts in the bone marrow; and/or
(g) is not a candidate for an HSCT; and/or
(h) is not intended to or set to receive an HSCT; and/or
(i) has a HSCT-naive status; and/or
(j) has a naive status for another treatment or therapy for the disease or condition.

3. The cells for use of claim 1 or 2, wherein the disease or condition is a tumor or a cancer.

4. The cells for use of any one of claims 1-3, wherein the subject exhibits morphologic disease, minimal disease, has relapsed, is refractory or non-responsive to a prior therapy or therapeutic agent for treating the disease or condition at the time of or immediately prior to the initial administration of the antigen-receptor expressing cells.

5. The cells for use of any one of claims 1-4, wherein the disease or condition is
(i) selected from leukemia, lymphoma, chronic lymphocytic leukemia (CLL), acutelymphoblastic leukemia (ALL), non-Hodgkin's lymphoma, acute myeloid leukemia, multiple myeloma, refractory follicular lymphoma, mantle cell lymphoma, indolent B cell lymphoma, B cell malignancies, cancers of the colon, lung, liver, breast, prostate, ovarian, skin, melanoma, bone, and brain cancer, ovarian cancer, epithelial cancers, renal cell carcinoma, pancreatic adenocarcinoma, Hodgkin's lymphoma, cervical carcinoma, colorectal cancer, glioblastoma, neuroblastoma, Ewing sarcoma, medulloblastoma, osteosarcoma, synovial sarcoma, mesothelioma, and combinations thereof; or
(ii) a blood cancer, which is optionally leukemia, which is optionally (aa) acute lymphoblastic leukemia (ALL), which is optionally B cell-derived ALL, or (bb) a chronic lymphocyte leukemia (CLL), which is optionally B cell-derived CLL, or (cc) acute myeloid leukemia (AML), which is optionally B cell-derived AML; or
(ii) lymphoma, which is optionally non-Hodgkin lymphoma (NHL), which is optionally B cell-derived NHL

6. The method of any one of claims 1-5, wherein the HSCT is allogeneic to the subject.

7. The cells for use of any one of claims 1-6, wherein the antigen receptor specifically binds to an antigen expressed by a cell or tissue of the disease or condition or associated with the disease or condition.

8. The cells for use of any one of claims 1-7, wherein the antigen receptor is a T cell receptor (TCR) or a functional non-TCR.

9. The cells for use of any one of claims 1-7, wherein the antigen receptor is a chimeric antigen receptor (CAR).

10. The cells for use of claim 9, wherein the CAR comprises an extracellular antigenrecognition domain that specifically binds to the antigen and an intracellular signaling domain comprising an ITAM, and optionally a costimulatory signaling region, optionally wherein (a) the antigen comprises CD19, and/or (b) the intracellular signaling domain comprises an intracellular domain of a CD3-zeta (CD3ζ) chain, and/or (c) the costimulatory signaling region comprises a signaling domain of a CD28 or a signaling domain of a 4-1BB and optionally a transmembrane and/or extracellular portion of a CD28

11. The cells for use of any one of claims 1-10, wherein the cells comprise or are T cells, optionally wherein the T cells are autologous to the subject.

12. The cells for use of claim 11, wherein the T cells selected from CD4⁺ T cell, CD8⁺ T cells, and combinations thereof, optionally wherein the T cells comprise CD4⁺ T cell and CD8⁺ T cells, optionally wherein the ratio of CD4⁺T cells to CD8⁺ T cells is between about 1:5 and about 5:1 or between about 1:2 and about 2:1.

13. The cells for use of any one of claims 1-12, wherein the dose of cells comprises between about 0.2 × 10⁶ cells/kg body weight of the subject and about 6 × 10⁶ cells/kg, about 0.5 × 10⁶ cells/kg body weight of the subject and about 3 × 10⁶ cells/kg, between about 0.75 × 10⁶ cells/kg and about 2.5 × 10⁶ cells/kg or between about 1 × 10⁶ cells/kg and about 2 × 10⁶ cells/kg, each inclusive.

14. The cells for use of any one of claims 1-13, wherein the cells of the dose are administered in a single pharmaceutical composition comprising the cells or in a plurality of compositions, collectively comprising the cells of the dose, over a period of no more than three days.

15. The cells for use of any one of claims 1-14, wherein the subject is administered a consecutive dose of cells expressing a recombinant antigen receptor after administering the first dose of the antigen receptor-expressing cells, optionally wherein
(a) the consecutive dose is administered at a time point that is between about 9 and about 35 days, between about 14 and about 28 days, between about 15 and about 27 days or is between about 17 days and about 21 days, each inclusive, after the initial administration of the first dose; and/or
(b) the antigen receptor expressed by the cells in the consecutive dose (i) is identical or substantially identical to the antigen receptor expressed by cells in the first dose, or (ii) binds to the same antigen as the antigen specifically bound by the antigen receptor expressed by cells of the first dose; and/or
(c) the consecutive dose is an amount sufficient for reduction in burden of a disease or condition in the subject; and/or
(d) the consecutive dose comprises less than or about the same number of antigen receptor-expressing cells as the number of the antigen receptor-expressing cells in the first dose, optionally wherein the subject exhibits morphologic disease after administration of the first dose and before the initial administration of the consecutive dose; and/or
(e) the consecutive dose comprises an increased number of antigen receptor-expressing cells as compared to the first dose, optionally wherein the increased number is at least about 2-fold, about 5-fold, or about 10-fold greater than the number of the antigen receptor-expressing cells in the first dose, and optionally wherein the subject exhibits minimal disease after administration of the first dose and before the initial administration of the consecutive dose.
